# EUROPEAN PATENT SPECIFICATION

(11) **EP 2 875 353 B1**
(45) Date of publication and mention of the grant of the patent: **14.09.2016**
(21) Application number: 13739591.9
(22) Date of filing: 12.07.2013
(51) Int. Cl.: G01N 33/53

(54) **METHODS OF DETECTING DNA, RNA AND PROTEIN IN BIOLOGICAL SAMPLES**
VERFAHREN ZUM NACHWEIS VON DNA, RNA UND PROTEINEN IN BIOLOGISCHEN PROBEN
PROCÉDÉS DE DÉTECTION D'ADN, D'ARN ET D'UNE PROTÉINE DANS DES ÉCHANTILLONS BIOLOGIQUES

(30) Priority: 17.07.2012 US 201213551190
(43) Date of publication of application: 27.05.2015
(73) Proprietor: General Electric Company, Schenectady, NY 12345 (US)
(72) Inventor: SOOD, Anup, Niskayuna, New York 12309 (US); GAO, Wei, Niskayuna, New York 12309 (US); GERDES, Michael, John, Niksayuna, New York 12309 (US); GINTY, Fiona Mary, Niskayuna, New York 12309 (US); SEPPO, Antti, Eljas, Niksayuna, New York 12309 (US); COLLINS, Elizabeth Mary, Niskayuna, New York 12309 (US)
(74) Representative: Bannan, Sally
(86) International application number: PCT/US2013/050218
(87) International publication number: WO 2014/014754

(56) References cited:
- GENDELMAN H E ET AL: "A double labeling technique for performing immunocytochemistry and in situ hybridization in virus infected cell cultures and tissues", JOURNAL OF VIROLOGICAL METHODS, ELSEVIER BV, NL, vol. 11, no. 2, 1 June 1985 (1985-06-01), pages 93-103, XP023696206, ISSN: 0166-0934, DOI: 10.1016/0166-0934(85)90033-3 [retrieved on 1985-06-01]
- OHSHIMA KOICHI ET AL: "Detection of human T lymphotrophic virus type I (HTLV-I) DNA and mRNA in individual cells by polymerase chain reaction (PCR) in situ hybridization (ISH) and reverse transcription (RT)-PCR ISH", HEMATOLOGICAL ONCOLOGY, WILEY, CHICHESTER, US, vol. 14, no. 2, 1 June 1996 (1996-06-01), pages 91-100, XP009173374, ISSN: 0278-0232 [retrieved on 1998-12-04]
- PERRIN A ET AL: "A combined oligonucleotide and protein microarray for the codetection of nucleic acids and antibodies associated with human immunodeficiency virus, hepatitis B virus, and hepatitis C virus infections", ANALYTICAL BIOCHEMISTRY, ACADEMIC PRESS INC, NEW YORK, vol. 322, no. 2, 15 November 2003 (2003-11-15), pages 148-155, XP004468598, ISSN: 0003-2697, DOI: 10.1016/J.AB.2003.08.002
- KUMAR BEENA ET AL: "Tissue microarrays: a practical guide", PATHOLOGY, MODERN MEDICINE, SYDNEY, AU, vol. 36, no. 4, 1 August 2004 (2004-08-01) , pages 295-300, XP009173355, ISSN: 0031-3025

## Description

### BACKGROUND

Various methods may be used in biology and in medicine to observe different targets in a biological sample. For example, analysis of proteins in histological sections and other cytological preparations may be performed using the techniques of histochemistry, immunohistochemistry (IHC), or immunofluorescence.

Methods of iteratively analyzing an individual sample are described in U.S. Patent No. 7,629,125 and U.S. Patent No. 7,741,046. In particular, U.S. Patent No.7,741,046 provides methods of detecting multiple targets in a biological sample that involves the use of oxidation for inactivating signal generators (e.g., for bleaching fluorescent dyes.).

In situ detection of proteins and DNA targets is routinely used as a diagnostic tool in cancer management. In recent years methods have been developed to look at these targets together in the same tissue section to determine correlations of these markers to each other and to clinical parameters. Another important cellular target is RNA. A variety of different RNA types have been identified and in addition to acting as templates for protein synthesis, a number of these, e.g. miRNA, control gene expression and hence cellular function and/or disease progression. RNA stability presents a unique challenge and extreme precautions are generally required to prevent RNase contamination. Therefore it is advisable to perform detection of RNA early in the process. Current methods to perform RNA detection in formalin fixed paraffin embedded tissue, however, have generally been performed after extensive protease treatment, which is incompatible with downstream detection of protein targets.

Disclosed herein are methods to detect RNA species without protease treatment and the simultaneous detection of the three types of targets; protiens, DNA, and RNA in the same sample. Each target plays a significant role is normal cellular function as well as disease progression and requires specific sample preparation that is not necessarily compatible with all targets. In situ detection in the same sample will allow better correlations between the expression of these different targets and better analysis of their relationship to disease.

### BRIEF DESCRIPTION

Disclosed herein are novel methods of probing multiple targets in a biological sample wherby the targets are DNA, RNA and protein.

In some embodiments, a method of probing multiple targets in a biological sample comprising a number of steps is disclosed. The steps include subjecting the sample to an in situ hybridization reaction using a labeled nucleic acid probe that directly or indirectly binds an RNA target, observing a signal from the labeled probe bound to the RNA target, and optionally removing the signal from the labeled probe. The method further comprises the steps of subjecting the sample to an antigen retrieval protocol to retrieve the sample's protein epitopes, subjecting the sample to an in situ hybridization reaction using an antibody-based method and attaching one or more antibody probe to antigens on the sample, observing a signal from the one or more antibody probes, removing the signal from the antibody probes, optionally applying a protease treatment to access the sample's DNA targets, subjecting the sample to an in situ hybridization reaction using a labeled nucleic acid probe to directly or indirectly label one or more of the sample's DNA targets, observing a signal from the labeled DNA targets, and optionally removing the signal from the one or more labeled DNA targets.

In some embodiments, the methods further comprise staining the sample with one or more control probes to allow for registration of multiple images of the sample and optionally registering multiple images of the sample. Still other embodiments include the method of analyzing the expression of protein, RNA and DNA from the multiple images.

### DESCRIPTION OF THE FIGURES

FIG. 1 is a schematic representation of a method of probing multiple targets in a biological sample wherein the targets comprise RNA, DNA, and protein.
FIG. 2a shows multiplex RNA, protein and DNA staining of a grade II lung squamous cell carcinomaA: cell nuclei stained with DAPI, B: U6 RNA, C: EGFR, D: Cytokeratin 7, E: IGF1R, F: NaKATPase, G: cMET and H: EGFR.
FIG. 2b, panels I & J, are zoomed in sections of same fields of view of panels G & H in FIG 2a, : no significant cMET staining was observed.
FIG. 3a shows multiplex RNA, protein and DNA staining of a lung metastatic adenocarcinoma. A: cell nuclei stained with *DAPI, B: U6 RNA, C: EGFR, D: Cytokeratin 7, E: IGF1R, F: NaKATPase, G: cMET and H: EGFR.*
FIG. 3b, panels I & J, are zoomed in sections of same fields of view of panels G & H in FIG. 3a.

### DETAILED DESCRIPTION

To more clearly and concisely describe and point out the subject matter of the claimed invention, the following definitions are provided for specific terms, which are used in the following description and the appended claims.

The singular forms "a" "an" and "the" include plural referents unless the context clearly dictates otherwise. Approximating language, as used herein throughout the specification and claims, may be applied to modify any quantitative representation that could permissibly vary without resulting in a change in the basic function to which it is related. Accordingly, a value modified by a term such as "about" is not to be limited to the precise value specified. Unless otherwise indicated, all numbers expressing quantities of ingredients, properties such as molecular weight, reaction conditions, so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least each numerical parameter should at least be construed in light of the number of reported significant digits and by applying ordinary rounding techniques.

As used herein, the term "antibody" refers to an immunoglobulin that specifically binds to and is thereby defined as complementary with a particular spatial and polar organization of another molecule. The antibody may be monoclonal or polyclonal and may be prepared by techniques that are well known in the art such as immunization of a host and collection of sera (polyclonal), or by preparing continuous hybrid cell lines and collecting the secreted protein (monoclonal), or by cloning and expressing nucleotide sequences or mutagenized versions thereof, coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM. Functional antibody fragments may include portions of an antibody capable of retaining binding at similar affinity to full-length antibody (for example, Fab, Fv and F(ab')₂, or Fab'). In addition, aggregates, polymers, and conjugates of immunoglobulins or their fragments may be used where appropriate so long as binding affinity for a particular molecule is substantially maintained.

As used herein, the term "binder" refers to a molecule that may bind to one or more targets in the biological sample. A binder may specifically bind to a target. Suitable binders may include one or more of natural or modified peptides, proteins (e.g., antibodies, affibodies, or aptamers), nucleic acids (e.g., polynucleotides, DNA, RNA, or aptamers); polysaccharides (e.g., lectins, sugars), lipids, enzymes, enzyme substrates or inhibitors, ligands, receptors, antigens, or haptens. A suitable binder may be selected depending on the sample to be analyzed and the targets available for detection. For example, a target in the sample may include a ligand and the binder may include a receptor or a target may include a receptor and the binder may include a ligand. Similarly, a target may include an antigen and the binder may include an antibody or antibody fragment or vice versa. In some embodiments, a target may include a nucleic acid and the binder may include a complementary nucleic acid. In some embodiments, both the target and the binder may include proteins capable of binding to each other.

As used herein, the term "biological sample" refers to a sample obtained from a biological subject, including sample of biological tissue or fluid origin obtained *in vivo* or *in vitro.* Such samples can be, but are not limited to, body fluid (e.g., blood, blood plasma, serum, or urine), organs, tissues, fractions, and cells isolated from mammals including, humans. Biological samples also may include sections of the biological sample including tissues (e.g., sectional portions of an organ or tissue). Biological samples may also include extracts from a biological sample, for example, an antigen from a biological fluid (e.g., blood or urine).

A biological sample may be of prokaryotic origin or eukaryotic origin (e.g., insects, protozoa, birds, fish, reptiles). In some embodiments, the biological sample is mammalian (e.g., rat, mouse, cow, dog, donkey, guinea pig, or rabbit). In certain embodiments, the biological sample is of primate origin (e.g., example, chimpanzee, or human).

As used herein, the term "probe" refers to an agent having a binder and a label, such as a signal generator or an enzyme. In some embodiments, the binder and the label (signal generator or the enzyme) are embodied in a single entity. The binder and the label may be attached directly (e.g., via a fluorescent molecule incorporated into the binder) or indirectly (e.g., through a linker, which may include a cleavage site) and applied to the biological sample in a single step. In alternative embodiments, the binder and the label are embodied in discrete entities (e.g., a primary antibody capable of binding a target and an enzyme or a signal generator-labeled secondary antibody capable of binding the primary antibody). When the binder and the label (signal generator or the enzyme) are separate entities they may be applied to a biological sample in a single step or multiple steps. As used herein, the term "fluorescent probe" refers to an agent having a binder coupled to a fluorescent signal generator.

As used herein, the term "signal generator" refers to a molecule capable of providing a detectable signal using one or more detection techniques (e.g., spectrometry, calorimetry, spectroscopy, or visual inspection). Suitable examples of a detectable signal may include an optical signal, and electrical signal, or a radioactive signal. Examples of signal generators include one or more of a chromophore, a fluorophore, a Raman-active tag, or a radioactive label. As stated above, with regard to the probe, the signal generator and the binder may be present in a single entity (e.g., a target binding protein with a fluorescent label) in some embodiments. Alternatively, the binder and the signal generator may be discrete entities (e.g., a receptor protein and a labeled-antibody against that particular receptor protein) that associate with each other before or upon introduction to the sample.

As used herein, the term "control probe" refers to an agent having a binder coupled to a signal generator or a signal generator capable of staining directly, such that the signal generator retains at least 80 percent signal after contact with a solution of an signal inactivation agent employed to inactivate the fluorescent probe. A suitable signal generator in a control probe is not substantially inactivated when contacted with the signal inactivation agent. Suitable examples of signal generators may include a radioactive label or a non-oxidizable fluorophore (e.g., DAPI)

As used herein, the term "enzyme" refers to a protein molecule that can catalyze a chemical reaction of a substrate. In some embodiments, a suitable enzyme catalyzes a chemical reaction of the substrate to form a reaction product that can bind to a receptor (e.g., phenolic groups) present in the sample or a solid support to which the sample is bound. A receptor may be exogeneous (that is, a receptor extrinsically adhered to the sample or the solid-support) or endogeneous (receptors present intrinsically in the sample or the solid-support). Examples of suitable enzymes include peroxidases, oxidases, phosphatases, esterases, and glycosidases. Specific examples of suitable enzymes include horseradish peroxidase, alkaline phosphatase, β-D-galactosidase, lipase, and glucose oxidase.

As used herein, the term "enzyme substrate" refers to a chemical compound that is chemically catalyzed by an enzyme to form a reaction product. In some embodiments, the reaction product is capable of binding to a receptor present in the sample or a solid support to which the sample is bound. In some embodiments, enzyme substrates employed in the methods herein may include non-chromogenic or non-chemiluminescent substrates. A signal generator may be attached to the enzyme substrate as a label.

As used herein, the term "chromophore" refers to a part of a molecule where the energy difference between two different molecular orbitals falls within the range of the visible spectrum. A chromophore may be responsible for a color of the molecule effected by absorbance of certain wavelengths of visible light and transmittance or reflectance of other wavelengths.

As used herein, the term "fluorophore" or "fluorescent signal generator" refers to a chemical compound, which when excited by exposure to a particular wavelength of light, emits light at a different wavelength. Fluorophores may be described in terms of their emission profile, or "color." Green fluorophores (for example Cy3, FITC, and Oregon Green) may be characterized by their emission at wavelengths generally in the range of 515-540 nanometers. Red fluorophores (for example Texas Red, Cy5, and tetramethylrhodamine) may be characterized by their emission at wavelengths generally in the range of 590-690 nanometers. Examples of fluorophores include, but are not limited to, 4-acetamido-4'-isothiocyanatostilbene-2,2'disulfonic acid, acridine, derivatives of acridine and acridine isothiocyanate, 5-(2'-aminoethyl)aminonaphthalene-1-sulfonic acid (EDANS), 4-amino-N-[3-vinylsulfonyl)phenyl]naphthalimide-3,5 disulfonate (Lucifer Yellow VS), N-(4-anilino-1-naphthyl)maleimide, anthranilamide, Brilliant Yellow, coumarin, coumarin derivatives, 7-amino-4-methylcoumarin (AMC, Coumarin 120), 7-amino-trifluoromethylcouluarin (Coumaran 151), cyanosine; 4',6-diaminidino-2-phenylindole (DAPI), 5',5"-dibromopyrogallol-sulfonephthalein (Bromopyrogallol Red), 7-diethylamino-3-(4'-isothiocyanatophenyl)4-methylcoumarin, -, 4,4'-diisothiocyanatodihydro-stilbene-2,2'-disulfonic acid, 4, 4'-diisothiocyanatostilbene-2,2'-disulfonic acid, 5-[dimethylamino]naphthalene-1-sulfonyl chloride (DNS, dansyl chloride), eosin, derivatives of eosin such as eosin isothiocyanate, erythrosine, derivatives of erythrosine such as erythrosine B and erythrosin isothiocyanate; ethidium; fluorescein and derivatives such as 5-carboxyfluorescein (FAM), 5-(4,6-dichlorotriazin-2-yl) aminofluorescein (DTAF), 2'7'-dimethoxy-4'5'-dichloro-6-carboxyfluorescein (JOE), fluorescein, fluorescein isothiocyanate (FITC), QFITC (XRITC); fluorescamine derivative (fluorescent upon reaction with amines); IR144; IR1446; Malachite Green isothiocyanate; 4-methylumbelliferone; ortho cresolphthalein; nitrotyrosine; pararosaniline; Phenol Red, B-phycoerythrin; o-phthaldialdehyde derivative (fluorescent upon reaction with amines); pyrene and derivatives such as pyrene, pyrene butyrate and succinimidyl 1-pyrene butyrate; Reactive Red 4 (Cibacron .RTM. Brilliant Red 3B-A), rhodamine and derivatives such as 6-carboxy-X-rhodamine (ROX), 6-carboxyrhodamine (R6G), lissamine rhodamine B sulfonyl chloride, rhodamine (Rhod), rhodamine B, rhodamine 123, rhodamine X isothiocyanate, sulforhodamine B, sulforhodamine 101 and sulfonyl chloride derivative of sulforhodamine 101 (Texas Red); N,N,N',N'-tetramethyl-6-carboxyrhodamine (TAMRA); tetramethyl Rhodamine, tetramethyl rhodamine isothiocyanate (TRITC); riboflavin; rosolic acid and lathanide chelate derivatives, quantum dots, cyanines, pyrelium dyes, and squaraines.

As used herein, the term "*in situ*" generally refers to an event occurring in the original location, for example, in intact organ or tissue or in a representative segment of an organ or tissue. In some embodiments, *in situ* analysis of targets may be performed on cells derived from a variety of sources, including an organism, an organ, tissue sample, or a cell culture. *In situ* analysis provides contextual information that may be lost when the target is removed from its site of origin. Accordingly, *in situ* analysis of targets describes analysis of target-bound probe located within a whole cell or a tissue sample, whether the cell membrane is fully intact or partially intact where target-bound probe remains within the cell. Furthermore, the methods disclosed herein may be employed to analyze targets *in situ* in cell or tissue samples that are fixed or unfixed.

As used herein, the term signal inactivation agent refers to a chemical that can either directly inactivate the signal or inactivate the signal after irradiation of the sample in the presence of the inactivation agent.

As used herein, the terms "irradiation" or "irradiate" refer to act or process of exposing a sample or a solution to non-ionizing radiation. In some embodiments, the non-ionizing irradiation has wavelengths between 350 nm and 1.3 um. In preferred embodiments, the non-ionizing radiation is visible light of 400-700 nm in wavelength. Irradiation may be accomplished by exposing a sample or a solution to a radiation source, e.g., a lamp, capable of emitting radiation of a certain wavelength or a range of wavelengths. In some embodiments, a molecule capable of undergoing photoexcitation is photoexcited as a result of irradiation. In some embodiments, the molecule capable of undergoing photoexcitation is a signal generator, e.g., a fluorescent signal generator. In some embodiments, irradiation of a fluorescent signal generator initiates a photoreaction between the fluorescent signal generator and the signal inactivation agent. In some embodiments, irradiation initiates a photoreaction that substantially inactivates the signal generator by photoactivated chemical bleaching. In other embodiments the signal inactivation agent undergoes photoexcitation to generate a reactive moiety that reacts with the signal generator to inactivate the signal. Optical filters may be used to restrict irradiation of a sample or a solution to a particular wavelength or a range of wavelengths. In some embodiments, the optical filters may be used to restrict irradiation to a narrow range of wavelengths for selective photoexcitation of one or more molecules capable of undergoing photoexcitation. The term "selective photoexcitation" refers to an act or a process, whereby one or more molecules capable of undergoing photoexcitation are photoexcited in the presence of one or more other molecules capable of undergoing photoexcitation that remain in the ground electronic state after irradiation.

In some embodiments, the molecule capable of undergoing photoexcitation is a fluorescent dye, e.g., a cyanine dye. In one further embodiment, irradiation limited to a range of wavelengths between 620-680 nm is used for selective photoexcitation of a Cy5 dye. In alternative embodiments, irradiation of a sample at a specific wavelength may also be accomplished by using a laser.

As used herein, the term "peroxidase" refers to an enzyme class that catalyzes an oxidation reaction of an enzyme substrate along with an electron donor Examples of peroxidase enzymes include horseradish peroxidase, cytochrome C peroxidase, glutathione peroxidase, microperoxidase, myeloperoxidase, lactoperoxidase, or soybean peroxidase.

As used herein, the term "peroxidase substrate" refers to a chemical compound that is chemically catalyzed by peroxidase to form a reaction product. In some embodiments, peroxidase substrates employed in the methods herein may include non-chromogenic or non-chemiluminescent substrates. A fluorescent signal generator may be attached to the peroxidase substrate as a label.

As used herein, the term "bleaching", "chemical bleaching", "photoactivated chemical bleaching" or "photoinduced chemical bleaching" refers to an act or a process whereby a signal generated by a signal generator is modified in the course of a reaction. In certain embodiments, the signal generator is irreversibly modified.

In some embodiments, the signal is diminished or eliminated as a result of photoactivated chemical bleaching. In some embodiments, the signal generator is completely bleached, i.e., the signal intensity decreases by about 100%. In some embodiments, the signal is an optical signal, and the signal generator is an optical signal generator. As used herein, the term "photoexcitation" refers to an act or a process whereby a molecule transitions from a ground electronic state to an excited electronic state upon absorption of radiation energy, e.g. upon irradiation. Photoexcited molecules can participate in chemical reactions, e.g., in electron transfer reactions. In some embodiments, a molecule capable of undergoing photoexcitation is a signal generator, e.g., a fluorescent signal generator.

As used herein, the term "photoreaction" or a "photoinduced reaction" refers to a chemical reaction that is initiated and/or proceeds as a result of photoexcitation of at least one reactant. The reactants in a photoreaction may be an electron transfer reagent and a molecule capable of undergoing photoexcitation. In some embodiments, a photoreaction may involve an electron transfer from the electron transfer reagent to the molecule that has undergone photoexcitation, i.e., the photoexcited molecule. In alternative embodiments, a photoreaction may also involve an electron transfer from the molecule that has undergone photoexcitation to the electron transfer reagent. In some embodiments, the molecule capable of undergoing photoexcitation is a fluorescent signal generator, e.g., a fluorophore. In some embodiments, photoreaction results in irreversible modification of one or more components of the photoreaction. In some embodiments, photoreaction substantially inactivates the signal generator by photoactivated chemical bleaching.

In some embodiments, the photoreaction may involve intermolecular electron transfer between the electron transfer reagent and the photoexcited molecule, e.g., the electron transfer occurs when the linkage between the electron transfer reagent and the photoexcited molecule is transitory, forming just prior to the electron transfer and disconnecting after electron transfer.

In some embodiments, the photoreaction may involve intramolecular electron transfer between the electron transfer reagent and the photoexcited molecule, e.g. the electron transfer occurs when the electron transfer reagent and the photoexcited molecule have been linked together, e.g., by covalent or electrostatic interactions, prior to initiation of the electron transfer process. The photoreaction involving the intramolecular electron transfer can occur, e.g., when the molecule capable of undergoing photoexcitation and the electron transfer reagent carry opposite charges and form a complex held by electrostatic interactions. For example, a cationic dye, e.g., a cationic cyanine dye and triphenylbutyl borate anion may form a complex, wherein intramolecular electron transfer may occur between the cyanine and borate moieties upon irradiation. In other embodiments electron transfer process may be an intermolecular process.

As used herein, the term "solid support" refers to an article on which targets present in the biological sample may be immobilized and subsequently detected by the methods disclosed herein. Targets may be immobilized on the solid support by physical adsorption, by covalent bond formation, or by combinations thereof. A solid support may include a polymeric, a glass, or a metallic material. Examples of solid supports include a membrane, a microtiter plate, a bead, a filter, a test strip, a slide, a cover slip, and a test tube.

As used herein, the term "specific binding" refers to the specific recognition of one of two different molecules for the other compared to substantially less recognition of other molecules. The molecules may have areas on their surfaces or in cavities giving rise to specific recognition between the two molecules arising from one or more of electrostatic interactions, hydrogen bonding, or hydrophobic interactions. Specific binding examples include, but are not limited to, antibody-antigen interactions, enzyme-substrate interactions, polynucleotide interactions, and the like. In some embodiments, a binder molecule may have an intrinsic equilibrium association constant (KA) for the target no lower than about 105 M-1 under ambient conditions such as a pH of about 6 to about 8 and temperature ranging from about 0°C to about 37°C.

As used herein, the term "target," refers to the component of a biological sample that may be detected when present in the biological sample. The target may be any substance for which there exists a naturally occurring specific binder (e.g., an antibody), or for which a specific binder may be prepared (e.g., a small molecule binder or an aptamer). In general, a binder may bind to a target through one or more discrete chemical moieties of the target or a three-dimensional structural component of the target (e.g., 3D structures resulting from peptide folding). The target may include one or more of natural or modified peptides, proteins (e.g., antibodies, affibodies, or aptamers), nucleic acids (e.g., polynucleotides, DNA, RNA, or aptamers); polysaccharides (e.g., lectins or sugars), lipids, enzymes, enzyme substrates, ligands, receptors, antigens, or haptens. In some embodiments, targets may include proteins or nucleic acids.

The invention includes embodiments that relate generally to methods applicable in analytical, diagnostic, or prognostic applications such as analyte detection, histochemistry, immunohistochemistry, immunofluorescence, chromogenic in situ hybridization, or fluorescence in situ hybridization (FISH). In some embodiments, the methods disclosed herein may be particularly applicable in histochemistry, immunostaining, immunohistochemistry, immunoassays, or immunofluorescence. In some embodiments, the methods disclosed herein may be particularly applicable in immunoblotting techniques, for example, western blots or immunoassays such as enzyme-linked immunosorbent assays (ELISA).

The disclosed methods relate generally to detection of multiple targets in a single biological sample. In some embodiments, methods of detecting multiple targets in a single biological sample using the same detection channel are disclosed. The targets may be present on the surface of cells in suspension, on the surface of cytology smears, on the surface of histological sections, on the surface of cell arrays or cell lysate array. on the surface of solid supports (such as gels, blots, glass slides, beads, or ELISA platesThe methods disclosed herein may allow detection of a plurality of targets in the same biological sample with little or no effect on the integrity of the biological sample. Detecting the targets in the same biological sample may further provide spatial information about the targets in the biological sample. Methods disclosed herein may also be applicable in analytical applications where a limited amount of biological sample may be available for analysis and the same sample may have to be processed for multiple analyses. Methods disclosed herein may also facilitate multiple analyses of solid-state samples (e.g., tissue sections) or samples adhered to a solid support (e.g., blots) without substantially stripping the targets. Furthermore, the same detection channel may be employed for detection of different targets in the sample, enabling fewer chemistry requirements for analyses of multiple targets. The methods may further facilitate analyses based on detection methods that may be limited in the number of simultaneously detectable targets because of limitations of resolvable signals. For example, using fluorescent-based detection, the number of targets that may be simultaneously detected may be limited to about four as only about four fluorescent signals may be resolvable based on their excitation and emission wavelength properties. In some embodiments, the methods disclosed herein may allow detection of greater than four targets using fluorescent-based detection system.

In some embodiments, the method of detecting DNA, RNA, and protein targets in a biological sample includes sequential detection of targets in the biological sample. The method generally includes the steps of detecting a first target in the biological sample, modifying the signal from the first target using a chemical agent, and detecting a second target in the biological sample. The method may further include repeating the step of modification of signal from the second target followed by detecting a third target in the biological sample, and so forth.

In certain embodiments, the biological sample may be adhered to a solid support or be in suspension such as, but not limited to, a hematopoetic cell or circulating tumor cell in a biolical fluid including a blood sample. As such in certain embodiments, detecting DNA, RNA, and protein targets in a biological sample includes sequential detection of targets in the biological sample wherein the biological sample is in suspension; for example an in situ hypridization reaction in solution.

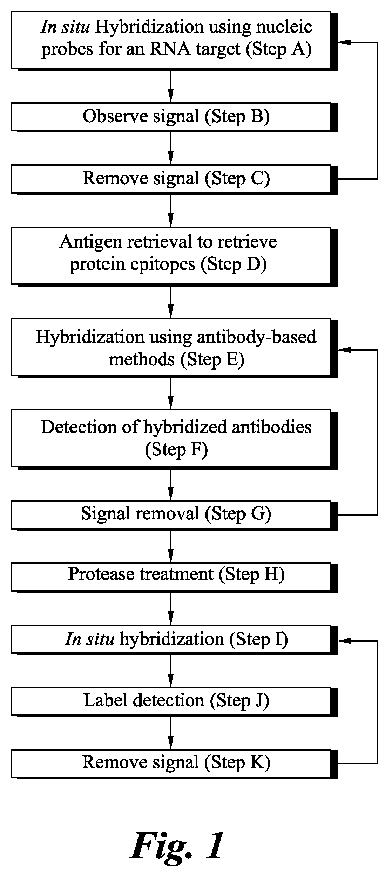

FIG. 1 is a schematic representation of one embodiment of the method wherein a biological sample is prepared from a paraffin or frozen section of a biological sample and subjected to in situ hybridization using one or more specifically labeled nucleic acid probes for an RNA target (step A) This is followed by step B, observation of a signal from the labels attached, directly or indirectly to the probes, and optionally step C removal of the signal from the RNA probes if multiple species of RNA is detected in multiple cycles by repeating steps A-C.

As shown further in FIG 1, the sample may then be subjected to antigen retrieval and detectionThis is shown in step D whereby the sample may be subject to an antigen retrieval protocal to retrieve protein epitopesAntigen retrieval may include, but is not limited to heat-induced methods or proteolytic digestion.

In certain embodimeents this is followed by step E, hybridization using antibody-based methods to target and attach an antibody probe to the anitgen. This may also result in removing of signals from the RNA probe. In certain embodiments, standard immunohistochemistry (IHC) or immunofluorescence (IF) techniques may be used. Once hybirdization is complete, detection of hybridized antibodies occurs by direct or indirect methods (step F), followed by removal of the signal from the antibody probes (step G). Steps E-G may be repeated a multiple times to detect multiple proteins.

Protease treatment is then applied (step H) to reveal or access, DNA targetsfollowed by *in situ* hybridization methods (ISH) to attach and target the DNA (step I), and detection of the labels attached, directly or indirectly to the probes (step J). In certain embodiments, an additional (step K) removing the signal either by signal inactivation or probe stripping may be performed if additional DNA targets are to be detected. In certain embodiments, chromogenic detection may be used.

In certain embodiments, after protease treatment, the step of preserving tissue morphology, prehybirdization or similar treatment steps may be applied.

In certain embodiments, a step for staining the sample with one or more control probes such as a morphological stain, e.g. DAPI may be added. The control probe may be applied one time or multiple times, to the sample. In certain embodiments, this may allow for registration of multiple images based on a morphological marker such that one or more composite images of the sample with the detected biomarkers may be obtained.

In certain embodiements, steps A, B, and C, steps E, F and G, and steps I, J and K may be repeated multiple times.

In certain embodiments the biological sample may contain multiple targets adhered to a solid support In some embodiments, a biological sample may include a tissue sample, a whole cell, a cell constituent, a cytospin, or a cell smear. In some embodiments, a biological sample essentially includes a tissue sample or tissue components. A tissue sample may include a collection of similar cells obtained from a tissue of a biological subject that may have a similar function. In some embodiments, a tissue sample may include a collection of similar cells obtained from a tissue of a human. Suitable examples of human tissues include, but are not limited to, (1) epithelium; (2) the connective tissues, including blood vessels, bone and cartilage; (3) muscle tissue; and (4) nerve tissue. The source of the tissue sample may be solid tissue obtained from a fresh, frozen and/or preserved organ or tissue sample or biopsy or aspirate; blood or any blood constituents; bodily fluids such as cerebral spinal fluid, amniotic fluid, peritoneal fluid, or interstitial fluid; or cells from any time in gestation or development of the subject. In some embodiments, the tissue sample may include primary or cultured cells, circulating disease or normal cells for example circulating tumor cells, activated leukocytes responding to an infectious agent, or cell lines.

In some embodiments, a biological sample includes tissue sections from healthy or diseased tissue samples (e.g., tissue section from colon, breast tissue, prostate). A tissue section may include a single part or piece of a tissue sample, for example, a thin slice of tissue or cells cut from a tissue sample. In some embodiments, multiple sections of tissue samples may be taken, e.g. a tissue microarray, and subjected to analysis, provided the methods disclosed herein may be used for analysis of the same section of the tissue sample with respect to at least three different types of targets (at molecular level, e.g. an RNA, a protein and a DNA). In some embodiments, the same section of tissue sample may be analyzed with respect to at least four different targets (at morphological or molecular level). In some embodiments, the same section of tissue sample may be analyzed with respect to greater than four different targets (at morphological or molecular level). In some embodiments, the same section of tissue sample may be analyzed at both morphological and molecular levels.

A tissue section, if employed as a biological sample may have a thickness in a range that is less than about 100 micrometers, in a range that is less than about 50 micrometers, in a range that is less than about 25 micrometers, or in range that is less than about 10 micrometers.

In some embodiments, a biological sample or the targets in the biological sample may be adhered to a solid support. A solid support may include microarrays (e.g., DNA or RNA microarrays), gels, blots, glass slides, beads, or ELISA plates. In some embodiments, a biological sample or the targets in the biological sample may be adhered to a membrane selected from nylon, nitrocellulose, and polyvinylidene difluoride. In some embodiments, the solid support may include a plastic surface selected from polystyrene, polycarbonate, and polypropylene.

A biological sample in accordance with one embodiment of the invention may be solid or fluid. Suitable examples of biological samples may include, but are not limited to, cultures, blood, plasma, serum, saliva, cerebral spinal fluid, pleural fluid, milk, lymph, sputum, semen, urine, stool, tears, saliva, needle aspirates, external sections of the skin, respiratory, intestinal, and genitourinary tracts, tumors, organs, cell cultures or cell culture constituents, or solid tissue sections. In some embodiments, the biological sample may be analyzed as is, that is, without harvest and/or isolation of the target of interest.

A biological sample may include any of the aforementioned samples regardless of their physical condition, such as, but not limited to, being frozen or stained or otherwise treated. In some embodiments, a biological sample may include compounds which are not naturally intermixed with the sample in nature such as preservatives, anticoagulants, buffers, fixatives, nutrients, antibiotics, or the like.

The sample may be a frozen tissue section or a paraffin embedded sample.. Parrafin samples refer to those samples wherein the biological sample has been previously fixed, for example in paraformaldyde followed by embedding in wax. In some embodiments, the tissue sample may be first fixed and then dehydrated through an ascending series of alcohols, infiltrated and embedded with paraffin or other sectioning media so that the tissue sample may be sectioned. In an alternative embodiment, a tissue sample may be sectioned and subsequently fixed. In some embodiments, the tissue sample may be embedded and processed in paraffin. Examples of paraffin that may be used include, but are not limited to, Paraplast, Broloid, and Tissuemay. Once the tissue sample is embedded, the sample may be sectioned by a microtome into sections that may have a thickness in a range of from about three microns to about five microns. Once sectioned, the sections may be attached to slides using adhesives. Examples of slide adhesives may include, but are not limited to, silane, gelatin, poly-L-lysine. In embodiments, if paraffin is used as the embedding material, the tissue sections may be deparaffinized and rehydrated in water. The tissue sections may be deparaffinized, for example, by using organic agents ,such as, xylenes and gradually descending series of alcohols,or detergents.

In some embodiments, aside from the sample preparation procedures discussed above, the tissue section may be subjected to further treatment prior to, during, or following in situ hybridization and/or immunohistochemistry. For example, in some embodiments, the tissue section may be subjected to epitope retrieval methods, such as, heating of the tissue sample in citrate buffer. In some embodiments, a tissue section may be optionally subjected to a blocking step to minimize any non-specific binding.

In some embodiments, the biological sample or a portion of the biological sample, or targets present in the biological sample (such as cell lysate) may be adhered on the surface of solid supports (such as gels, blots, glass slides, beads, or ELISA plates). In some embodiments, targets present in the biological sample may be adhered on the surface of solid supports. Targets in the biological sample may be adhered on the solid support by physical bond formation, by covalent bond formation, or both.

. Suitability of targets to be analyzed may be determined by the type and nature of analysis required for the biological sample. In some embodiments, a target may provide information about the presence or absence of an analyte in the biological sample. In another embodiment, a target may provide information on a state of a biological sample. For example, if the biological sample includes a tissue sample, the methods disclosed herein may be used to detect targets that may help in comparing different types of cells or tissues, comparing different developmental stages, detecting the presence of a disease or abnormality, or determining the type of disease or abnormality.

In certain embodiments, the targets in the biological sample may include one or more of peptides, proteins (e.g., antibodies, affibodies, or aptamers), nucleic acids (e.g., polynucleotides, DNA, RNA, or aptamers); polysaccharides (e.g., lectins or sugars), lipids, enzymes, enzyme substrates, ligands, receptors, antigens, or haptens. In some embodiments, targets may essentially include proteins or nucleic acids. One or more of the aforementioned targets may be characteristic of particular cells, while other targets may be associated with a particular disease or condition. In some embodiments, targets that may be detected and analyzed using the methods disclosed herein may include, but are not limited to, prognostic targets, hormone or hormone receptor targets, lymphoid targets, tumor targets, cell cycle associated targets, neural tissue and tumor targets, or cluster differentiation targets

Suitable examples of prognostic targets may include enzymatic targets such as galactosyl transferase II, neuron specific enolase, proton ATPase-2, or acid phosphatase.

Suitable examples of hormone or hormone receptor targets may include human chorionic gonadotropin (HCG), adrenocorticotropic hormone, carcinoembryonic antigen (CEA), prostate-specific antigen (PSA), estrogen receptor, progesterone receptor, androgen receptor, gC1q-R/p33 complement receptor, IL-2 receptor, p75 neurotrophin receptor, PTH receptor, thyroid hormone receptor, or insulin receptor.

Suitable examples of lymphoid targets may include alpha-1-antichymotrypsin, alpha-1-antitrypsin, B cell target, bcl-2, bcl-6, B lymphocyte antigen 36 kD, BM1 (myeloid target), BM2 (myeloid target), galectin-3, granzyme B, HLA class I Antigen, HLA class II (DP) antigen, HLA class II (DQ) antigen, HLA class II (DR) antigen, human neutrophil defensins, immunoglobulin A, immunoglobulin D, immunoglobulin G, immunoglobulin M, kappa light chain, kappa light chain, lambda light chain, lymphocyte/histocyte antigen, macrophage target, muramidase (lysozyme), p80 anaplastic lymphoma kinase, plasma cell target, secretory leukocyte protease inhibitor, T cell antigen receptor (JOVI 1), T cell antigen receptor (JOVI 3), terminal deoxynucleotidyl transferase, or unclustered B cell target.

Suitable examples of tumour targets may include alpha fetoprotein, apolipoprotein D, BAG-1 (RAP46 protein), CA19-9 (sialyl lewisa), CA50 (carcinoma associated mucin antigen), CA125 (ovarian cancer antigen), CA242 (tumour associated mucin antigen), chromogranin A, clusterin (apolipoprotein J), epithelial membrane antigen, epithelial-related antigen, epithelial specific antigen, gross cystic disease fluid protein-15, hepatocyte specific antigen, heregulin, human gastric mucin, human milk fat globule, MAGE-1, matrix metalloproteinases, melan A, melanoma target (HMB45), mesothelin, metallothionein, microphthalmia transcription factor (MITF), Muc-1 core glycoprotein. Muc-1 glycoprotein, Muc-2 glycoprotein, Muc-5AC glycoprotein, Muc-6 glycoprotein, myeloperoxidase, Myf-3 (Rhabdomyosarcoma target), Myf-4 (Rhabdomyosarcoma target), MyoD1 (Rhabdomyosarcoma target), myoglobin, nm23 protein, placental alkaline phosphatase, prealbumin, prostate specific antigen, prostatic acid phosphatase, prostatic inhibin peptide, PTEN, renal cell carcinoma target, small intestinal mucinous antigen, tetranectin, thyroid transcription factor-1, tissue inhibitor of matrix metalloproteinase 1, tissue inhibitor of matrix metalloproteinase 2, tyrosinase, tyrosinase-related protein-1, villin, or von Willebrand factor.

Suitable examples of cell cycle associated targets may include apoptosis protease activating factor-1, bcl-w , bcl-x, bromodeoxyuridine, CAK (cdk-activating kinase), cellular apoptosis susceptibility protein (CAS), caspase 2, caspase 8, CPP32 (caspase-3), CPP32 (caspase-3), cyclin dependent kinases, cyclin A, cyclin B1, cyclin D1, cyclin D2, cyclin D3, cyclin E, cyclin G, DNA fragmentation factor (N-terminus), Fas (CD95), Fas-associated death domain protein, Fas ligand, Fen-1, IPO-38, Mcl-1, minichromosome maintenance proteins, mismatch repair protein (MSH2), poly (ADP-Ribose) polymerase, proliferating cell nuclear antigen, p16 protein, p27 protein, p34cdc2, p57 protein (Kip2), p105 protein, Stat 1 alpha, topoisomerase I, topoisomerase II alpha, topoisomerase III alpha, or topoisomerase II beta.

Suitable examples of neural tissue and tumor targets may include alpha B crystallin, alpha-internexin, alpha synuclein, amyloid precursor protein, beta amyloid, calbindin, choline acetyltransferase, excitatory amino acid transporter 1, GAP43, glial fibrillary acidic protein, glutamate receptor 2, myelin basic protein, nerve growth factor receptor (gp75), neuroblastoma target, neurofilament 68 kD, neurofilament 160 kD, neurofilament 200 kD, neuron specific enolase, nicotinic acetylcholine receptor alpha4, nicotinic acetylcholine receptor beta2, peripherin, protein gene product 9, S-100 protein, serotonin, SNAP-25, synapsin I, synaptophysin, tau, tryptophan hydroxylase, tyrosine hydroxylase, or ubiquitin.

Suitable examples of cluster differentiation targets may include CD1a, CD1b, CD1 c, CD1d, CD1e, CD2, CD3delta, CD3epsilon, CD3gamma, CD4, CD5, CD6, CD7, CD8alpha, CD8beta, CD9, CD10, CD11a, CD11b, CD11c, CDw12, CD13, CD14, CD15, CD15s, CD16a, CD16b, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD44R, CD45, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CDw75, CDw76, CD77, CD79a, CD79b, CD80, CD81, CD82, CD83, CD84, CD85, CD86, CD87, CD88, CD89, CD90, CD91, CDw92, CDw93, CD94, CD95, CD96, CD97, CD98, CD99, CD100, CD101, CD102, CD103, CD104, CD105, CD106, CD107a, CD107b, CDw108, CD109, CD114, CD115, CD116, CD117, CDw119 CD120a, CD120b, CD121a, CDw121b, CD122, CD123, CD124, CDw125, CD126, CD127, CDw128a, CDw128b, CD130, CDw131, CD132, CD134, CD135, CDw136, CDw137, CD138, CD139, CD140a, CD140b, CD141, CD142, CD143, CD144, CDw145, CD146, CD147, CD148, CDw149, CDw150, CD151, CD152, CD153, CD154, CD155, CD156, CD157, CD158a, CD158b, CD161, CD162, CD163, CD164, CD165, CD166, and TCR-zeta.

Other suitable prognostic targets may include centromere protein-F (CENP-F), giantin, involucrin, lamin A&C (XB 10), LAP-70, mucin, nuclear pore complex proteins, p180 lamellar body protein, ran, r, cathepsin D, Ps2 protein, Her2-neu, P53, S100, epithelial target antigen (EMA), TdT, MB2, MB3, PCNA, or Ki67.

The detection of RNA,in steps A, B, and C, generally involves an optional prehybridization step usually with salmon sperm DNA or tRNA for blocking followed by a hybridization step using sequence-specific probes to targets of interest at elevated temperature. In the absence of a prehybridization step, blocking agent is used with the probe itself during the hybridization step. Optimum probe concentration and temperature are generally empirically determined for best signal to noise ratio but are a function of probe Tm, buffer composition and probe type, e.g. LNA vs DNA backbones. Hybridization time can also vary significant from about an half an hour or less to overnight hybridization and can be controlled by probe concentration. Post hybridization sample are subjected to one or more stringent washes to remove excess and non-specifically bound probe. Finally the probe is detected either directly if a signal generator is directly attached to the probe or indirectly with or without signal amplification. Detection may occur using a variety of techniques, including but not limited to manual observation, film or other recording devise, cameras, video recordings or a combination thereof. In some embodiments, the signal may be removed by the methods discussed above by chemical inactivation and sample may be probed for additional RNA species. Alternatively in other embodiments where the next step is protein detection, signal may be removed during the antigen retrieval step by denaturation of the bound probe or inactivation of signal due to antigen retrieval process that involves high temperature heating in acid and/or base.

In certain embodiments, the aforementioned biological sample may then be subjected to antigen retrieval and detection. An antigen target may be present on the surface of a biological sample (for example, an antigen on a surface of a tissue section). In some embodiments, an antigen target may not be inherently present on the surface of a biological sample and the biological sample may have to be processed to make the target available on the surface (e.g., antigen recovery, enzymatic digestion or epitope retrieval).

In general, as shown in steps D through G, in certain embodiments after antigen retrieval antigens are subjected to hybridization with a binder as previously defined. In certain embodiments in the binder includes an antibody to bind to the antigen. A suitable antibody may include monoclonal antibodies, polyclonal antibodies, multispecific antibodies ,for example, bispecific antibodies,, or antibody fragments so long as they bind specifically to a target antigen. In some embodiments, the methods disclosed herein may be employed in immunohistochemistry (IHC). Immunochemistry may involve binding of a target antigen to an antibody-based binder to provide information about the tissues or cells (for example, diseased versus normal cells). Examples of antibodies (and the corresponding diseases/disease cells) suitable as binders for methods disclosed herein include, but are not limited to, anti-estrogen receptor antibody (breast cancer), anti-progesterone receptor antibody (breast cancer), anti-p53 antibody (multiple cancers), anti-Her-2/neu antibody (multiple cancers), anti-EGFR antibody (epidermal growth factor, multiple cancers), anti-cathepsin D antibody (breast and other cancers), anti-Bcl-2 antibody (apoptotic cells), anti-E-cadherin antibody, anti-CA125 antibody (ovarian and other cancers), anti-CA15-3 antibody (breast cancer), anti-CA19-9 antibody (colon cancer), anti-c-erbB-2 antibody, anti-P-glycoprotein antibody (MDR, multi-drug resistance), anti-CEA antibody (carcinoembryonic antigen), anti-retinoblastoma protein (Rb) antibody, anti-ras oneoprotein (p21) antibody, anti-Lewis X (also called CD15) antibody, anti-Ki-67 antibody (cellular proliferation), anti-PCNA (multiple cancers) antibody, anti-CD3 antibody (T-cells), anti-CD4 antibody (helper T cells), anti-CD5 antibody (T cells), anti-CD7 antibody (thymocytes, immature T cells, NK killer cells), anti-CD8 antibody (suppressor T cells), anti-CD9/p24 antibody (ALL), anti-CD10 (also called CALLA) antibody (common acute lymphoblasic leukemia), anti-CD11c antibody (Monocytes, granulocytes, AML), anti-CD13 antibody (myelomonocytic cells, AML), anti-CD14 antibody (mature monocytes, granulocytes), anti-CD15 antibody (Hodgkin's disease), anti-CD19 antibody (B cells), anti-CD20 antibody (B cells), anti-CD22 antibody (B cells), anti-CD23 antibody (activated B cells, CLL), anti-CD30 antibody (activated T and B cells, Hodgkin's disease), anti-CD31 antibody (angiogenesis marker), anti-CD33 antibody (myeloid cells, AML), anti-CD34 antibody (endothelial stem cells, stromal tumors), anti-CD35 antibody (dendritic cells), anti-CD38 antibody (plasma cells, activated T, B, and myeloid cells), anti-CD41 antibody (platelets, megakaryocytes), anti-LCA/CD45 antibody (leukocyte common antigen), anti-CD45RO antibody (helper, inducer T cells), anti-CD45RA antibody (B cells), anti-CD39, CD100 antibody, anti-CD95/Fas antibody (apoptosis), anti-CD99 antibody (Ewings Sarcoma marker, MIC2 gene product), anti-CD106 antibody (VCAM-1; activated endothelial cells), anti-ubiquitin antibody (Alzheimer's disease), anti-CD71 (transferrin receptor) antibody, anti-c-myc (oncoprotein and a hapten) antibody, anti-cytokeratins (transferrin receptor) antibody, anti-vimentins (endothelial cells) antibody (B and T cells), anti-HPV proteins (human papillomavirus) antibody, anti-kappa light chains antibody (B cell), anti-lambda light chains antibody (B cell), anti-melanosomes (HMB45) antibody (melanoma), anti-prostate specific antigen (PSA) antibody (prostate cancer), anti-S-100 antibody (melanoma, salvary, glial cells), anti-tau antigen antibody (Alzheimer's disease), anti-fibrin antibody (epithelial cells), anti-keratins antibody, anti-cytokeratin antibody (tumor), anti-alpha-catenin (cell membrane), or anti-Tn-antigen antibody (colon carcinoma, adenocarcinomas, and pancreatic cancer).

Other specific examples of suitable antibodies may include, but are not limited to, anti proliferating cell nuclear antigen, clone pc10 (Sigma Aldrich, P8825); anti smooth muscle alpha actin (SmA), clone 1A4 (Sigma, A2547); rabbit anti beta catenin (Sigma, C 2206); mouse anti pan cytokeratin, clone PCK-26 (Sigma, C1801); mouse anti estrogen receptor alpha, clone 1D5 (DAKO, M 7047); beta catenin antibody, clone 15B8 (Sigma, C 7738); goat anti vimentin (Sigma, V4630); androgen receptor clone AR441 (DAKO, M3562); Von Willebrand Factor 7, keratin 5, keratin 8/18, e-cadherin, Her2/neu, Estrogen receptor, p53, progesterone receptor, beta catenin; donkey anti-mouse (Jackson Immunoresearch, 715-166-150); or donkey anti rabbit (Jackson Immunoresearch, 711-166-152).

In certain embodiments, the antigen detection process may involve contacting a probe solution (e.g., labeled-antibody solution) with the biological sample for a sufficient period of time and under conditions suitable for binding of a binder to the target (e.g., antigen). In certain embodiments, two detection methods may be used: direct or indirect. In a direct detection, a signal generator-labeled primary antibody (e.g., fluorophore-labeled primary antibody or enzyme-labeled primary antibody) may be incubated with an antigen in the tissue sample or the membrane, which may be visualized without further antibody interaction. In an indirect detection, an unconjugated primary antibody may be incubated with an antigen and then a labeled secondary antibody may bind to the primary antibody. Signal amplification may occur as several secondary antibodies may react with different epitopes on the primary antibody. In some embodiments two or more (at most five) primary antibodies (from different species, labeled or unlabeled) may be contacted with the tissue sample. Unlabeled antibodies may be then contacted with the corresponding labeled secondary antibodies. In alternate embodiments, a primary antibody and specific binding ligand-receptor pairs (such as biotin-streptavidin) may be used. The primary antibody may be attached to one member of the pair (for example biotin) and the other member (for example streptavidin) may be labeled with a signal generator or an enzyme. The secondary antibody, avidin, streptavidin, or biotin may be each independently labeled with a signal generator or an enzyme.

In embodiments where the primary antibody or the secondary antibody may be conjugated to an enzymatic label, a fluorescent signal generator-coupled substrate may be added to provide visualization of the antigen. In some embodiments, the substrate and the fluorescent signal generator may be embodied in a single molecule and may be applied in a single step. In other embodiments, the substrate and the fluorescent signal generator may be distinct entities and may be applied in a single step or multiple steps.

An enzyme coupled to the binder may react with the substrate to catalyze a chemical reaction of the substrate to covalently bind the fluorescent signal generator-coupled substrate the biological sample. In some embodiments, an enzyme may include horseradish peroxidase and the substrate may include tyramine. Reaction of the horseradish peroxidase (HRP) with the tyramine substrate may cause the tyramine substrate to covalently bind to phenolic groups present in the sample. In embodiments employing enzyme-substrate conjugates, signal amplification may be attained as one enzyme may catalyze multiple substrate molecules. In some embodiments, methods disclosed herein may be employed to detect low abundance targets using indirect detection methods (e.g., using primary-secondary antibodies), using HRP-tyramide signal amplification methods, or combinations of both (e.g., indirect HRP-tyramide signal amplification methods).

In certain embodiments, incorporation of signal amplification techniques into the methods described and correspondingly of the corresponding signal amplification techniques may depend on the sensitivity required for a particular target and the number of steps involved in the protocol.

A signal from the signal generator may be detected using a variety of observation or detection systems. The nature of the detection system used may depend upon the nature of the signal generators used. The detection system may include an, a charge coupled device (CCD) detection system a fluorescent detection system, an electrical detection system, a photographic film detection system, a chemiluminescent detection system, an enzyme detection system, an optical detection system, a near field detection system, or a total internal reflection (TIR) detection system.

One or more of the aforementioned techniques may be used to observe one or more characteristics of a signal from a signal generator (coupled with a binder or coupled with an enzyme substrate). In some embodiments, signal intensity, signal wavelength, signal location, signal frequency, or signal shift may be determined using one or more of the aforementioned techniques. In some embodiments, one or more aforementioned characteristics of the signal may be observed, measured, and recorded.

In some embodiments, the observed signal is a fluorescent signal, and a probe bound to a target in a biological sample may include a signal generator that is a fluorophore. In some embodiments, the fluorescent signal may be measured by determining fluorescence wavelength or fluorescent intensity using a fluorescence detection system. In some embodiments, a signal may be observed in situ, that is, a signal may be observed directly from the signal generator associated through the binder to the target in the biological sample. In some embodiments, a signal from the signal generator may be analyzed within the biological sample, obviating the need for separate array-based detection systems.

In some embodiments, observing a signal may include capturing an image of the biological sample. In some embodiments, a microscope connected to an imaging device may be used as a detection system, in accordance with the methods disclosed herein. In some embodiments, a signal generator (such as, fluorophore) may be excited and the signal (such as, fluorescence signal) obtained may be observed and recorded in the form of a digital signal (for example, a digitalized image). The same procedure may be repeated for different signal generators (if present) that are bound in the sample using the appropriate fluorescence filters.

In some embodiments, multiple different types of signals may be observed in the same sample. For example, one target may be detected with a fluorescent probe and a second target in the same sample may be detected with a chromogenic probe.

In certain embodiments, removal of the signal from the antibody probes (step G) comprises contacting the biological sample with a chemical agent, capable of selectively modifying one or more signal generators. In certain embodiments the chemical agent is an oxidizing agent that substantially inactivates both the fluorescent signal generator and the enzyme. In some embodiments, a the chemical agent may essentially include a basic solution of an oxidizing agent.

In certain embodiments susceptibility of different signal generators to a chemical agent may depend, in part, to the concentration of the signal generator, temperature, or pH. For example, two different fluorophores may have different susceptibility to an oxidizing agent depending upon the concentration of the oxidizing agent.

A suitable oxidizing agent may be selected from peroxide, sodium periodate, or ozone. In some embodiments, a suitable oxidizing agent may include peroxide or a peroxide source and the basic solution may include hydrogen peroxide. The concentration of hydrogen peroxide in the basic solution may be selected to substantially oxidize the fluorescent signal generator in a predetermined period of time. In some embodiments, the concentration of hydrogen peroxide in the basic solution may be selected to substantially inactivate both the fluorescent signal generator and the enzyme in a given period of time.

In some embodiments, a basic solution may include hydrogen peroxide in an amount that is in a range of from about 0.5 volume percent to about 5 volume percent, in a range of from about 1 volume percent to about 4 volume percent, or in a range of from about 1.5 volume percent to about 3.5 volume percent. In some specific embodiments, a basic solution may include hydrogen peroxide in an amount that is in a range of about 3 volume percent.

In some embodiments, steps E, F, and G may be repeated multiple times; contacting the biological sample with a subsequent (e.g., second, third, etc.) probe, observing the signal, and bleaching of the signal generator. The binding, observing, and bleaching steps may be repeated iteratively multiple times using an nth probe capable of binding to additional targets to provide the user with information about a variety of targets using a variety of probes and/or signal generators. In embodiments where binders coupled to enzymes may be employed as probes, binding steps may further include reacting steps involving reaction of the enzyme with an enzyme substrate coupled to fluorescent signal generator.

In some embodiments steps E, F, and G may be repeated 1-150 times, preferably 5-100 times, or more preferabbly 5-60 times, In some embodiments, the series of steps may be repeated 25-30 times or more preferabbly 2- 10 times. In some embodiments, a series of probes may be contacted with the biological sample in a sequential manner to obtain a multiplexed analysis of the biological sample. In some embodiments, a series of probe sets , wherein a probe set may include a mixture of more than one probe targeting a single type of targets (e.g. different RNA targets or different protein or different DNA targets), may be contacted with the biological sample in a sequential manner to obtain a multiplexed analysis of the biological sample. In certain preferred embodiemnts the mixture inclues 2 to10 probes, and preferably 2-5 probes. Multiplexed analysis generally refers to analysis of multiple targets in a biological sample using the same detection mechanism.

In some embodiments, the components of a biological sample are not significantly modified after repeated cycles of signal removal, binding, reacting (if applicable), and signal observing steps. In some embodiments, the components of a biological sample are not significantly modified during the bleaching step. In some embodiments, the components of the biological sample that are not significantly modified during the signal removal step are targets. In some embodiments, more than 80% of targets are not significantly modified in the course of the signal removal step. In some embodiments, more than 95% of targets are not significantly modified.

After the antigen-detection process, in certain embodiments steps H, I, J allow for the detection of DNA. In general, the method involves treatment of the sample with protease. Treatment time may vary depending upon the sample, how it was prepared, e.g. type of fixative, length of fixation etc., temperature of protease digestion and concentration of the protease itself. After protease treatment both the probe, in a hybridization buffer, and the target within the sample may be denturated by heating and the probe is applied to the sample-. Alternatively, probe and target may be denatured together after the probe has been applied to the sample. Hybridization is generally allowed to proceed overnight, although probes that require shorter hybridization time have been developed and may reduce the time of hybridization to about 1h or less. Post hybridization, strigent washes may be applied to remove excess probe as well as non-specifically bound probe. Sample may be treated with a morphological stain to stain the nuclei prior to detection of probe and morphological stain signal. In some embodiments a prehybridization step may be performed. In other embodiments, post protease treatment sample may be subjected to a fixation step to preserve tissue morphology. Methods of in situ DNA detection are well known in the art and various variations of it are described by Volpi & Bridger in Biotechniques, 45:385-409, 2008.

In certain embodiments a nucleic-acid based binder may be used to bind with the DNA target. The nuclei-acid based binder may form a Watson-Crick bond with the nucleic acid target. In another embodiment, the nucleic acid binder may form a Hoogsteen bond with the nucleic acid target, thereby forming a triplex. A nucleic acid binder that binds by Hoogsteen binding may enter the major groove of a nucleic acid target and hybridizes with the bases located there. Suitable examples of the above binders may include molecules that recognize and bind to the minor and major grooves of nucleic acids (for example, some forms of antibiotics.) In certain embodiments, the nucleic acid binders may form both Watson-Crick and Hoogsteen bonds with the nucleic acid target (for example, bis PNA probes are capable of both Watson-Crick and Hoogsteen binding to a nucleic acid).

The length of nucleic acid binder may also determine the specificity of binding. The energetic cost of a single mismatch between the binder and the nucleic acid target may be relatively higher for shorter sequences than for longer ones. In some embodiments, hybridization of smaller nucleic acid binders may be more specific than the hybridization of longer nucleic acid probes, as the longer probes may be more amenable to mismatches and may continue to bind to the nucleic acid depending on the conditions. In certain embodiments, shorter binders may exhibit lower binding stability at a given temperature and salt concentration.

Binders that may exhibit greater stability to bind short sequences may be employed. For example in certain embodiments, bis PNA may be used. In some embodiments, the nucleic acid binder may have a length in range of from about 4 nucleotides to several kilo bases, preferably from 12- 1000 nucleotides, and more preferably from 12 to 400 nucleotides. , In some embodiments, the nucleic acid binder may have a length in a range that is greater than about 1000 nucleotides. Notwithstanding the length of the nucleic acid binder, all the nucleotide residues of the binder may not hybridize to complementary nucleotides in the nucleic acid target. For example, the binder may include 50 nucleotide residues in length, and only 25 of those nucleotide residues may hybridize to the nucleic acid target. In some embodiments, the nucleotide residues that may hybridize may be contiguous with each other. The nucleic acid binders may be single stranded or may include a secondary structure. In some embodiments, a biological sample may include a cell or a tissue sample and the biological sample may be subjected to in-situ hybridization (ISH) using a nucleic acid binder. In some embodiments, a tissue sample may be subjected to in situ hybridization in addition to immunohistochemistry (IHC) to obtain desired information from the sample.

In yet other embodiments, the method may further includes binding at least one control probe to one or more target in the sample. The method further includes observing a signal from a bound fluorescent probe and a control signal from the control probe. The bound fluorescent probe is exposed to an inactivating agent that substantially inactivates the fluorescent probe and not the control probe. The method further includes binding at least one subsequent fluorescent probe to one or more target present in the sample followed by observing a signal from the subsequent bound fluorescent probe.

A control probe may include a signal generator that is stable towards an inactivating agent or the signal generating properties of the signal generator are not substantially effected when contacted with the inactivating agent. A signal generator may include a radioisotope or a fluorophore which are stable to the inactivating agent. A suitable radioisotope may include P³²,H³, ¹⁴C, ¹²⁵I or ¹³¹I. A suitable fluorophore may include DAPI.

In some embodiment in the last probe hybridization and detection step signal generators may include one or more stable signal generators which may be detectable by various types of mass detecters, such as a stable metal isotopes or a non-bleachable chromogens.

In some embodiments, a suitable signal generator may be coupled to a binder to form a control probe. For example, a radioactive label may be coupled to an antibody to form a control probe and the antibody may bind to one or more target antigens present in the biological sample. In other embodiments, a suitable signal generator may be capable of binding to one more targets in the sample and also providing a detectable signal, which is stable in the presence of the inactivating agent. For example, a suitable control probe may be DAPI, which is capable of binding to nucleic acids in the sample and also capable of providing a fluorescent signal that is stable to the inactivating agent.

In some embodiments, a control probe may be employed in the methods disclosed herein to provide an indication of the stability of the targets to the iterative staining steps. For example, a control probe may be bonded to a known target in the sample and a signal from the control observed and quantified. The control signal may be then monitored during the iterative staining steps to provide an indication of the stability of the targets or binders to the inactivated agents. In some embodiments, a quantitative measure,for example the signal intensity, of the control signal may be monitored to quantify the amount of targets present in the sample after the iterative probing steps.

In certain embodiments, a control probe may be employed to obtain quantitative information of the sample of interest, for example concentration of targets in the sample or molecular weight of the targets in the sample. In certain embodiments a control target, having a known concentration or molecular weight, may be loaded along with the sample of interest in a blotting technique. A control probe may be bonded to the control target and a control signal observed. The control signal may be then correlated with the signals observed from the sample of interest.

In certain embodiments, a control probe may be employed to provide for co-registration of multiple molecular information ,obtained through the iterative probing steps, and morphological information obtained, for example using a morphological stain such as DAPI).

In some embodiments methods may include co-registration of multiple fluorescent images with the bright-field morphological images obtained, for example images obtained using H&E. In some embodiments, the probes employed in the iterative probing steps may not have common compartmental information that may be used to register with the H&E images. A control probe, such as a DAPI nuclear stain, may be employed to co-register the nucleus stained with hematoxylin in the bright-field images with the fluorescent images. The fluorescent images and the bright-field images may be co-registered using image registration algorithms that may be grouped in two categories: intensity-based and feature-based techniques.

In some embodiments, the biological sample may be contacted with a morphological stain before, during, or after the contacting step with the first probe or subsequent probe. A morphological stain may include a dye that may stain different cellular components, in order to facilitate identification of cell type or disease status. In some embodiments, the morphological stain may be readily distinguishable from the signal generators in the probes, that is, the stain may not emit signal that may overlap with signal from the probe. For example, for a fluorescent morphological stain, the signal from the morphological stain may not autofluoresce in the same wavelength as the fluorophores used in the probes.

A morphological stain may be contacted with the biological sample before, during, or after, any one of the aforementioned steps. In certain embodiments, a morphological stain may be contacted with biological sample along with the first probe contact step. In some embodiments, a morphological stain may be contacted with the biological sample before contacting the sample with a chemical agent and after binding the first probe to the target. In some embodiments, a morphological stain may be contacted with a biological sample after contacting the sample with a chemical agent and modifying the signal.

In still other embodiments, a morphological stain may be contacted with a biological sample along with the second probe contact step. In some embodiments, a biological sample may be contacted with the morphological stain after binding the second probe to the target. In some embodiments, where the morphological stains may result in background noise for the fluorescent signal from the signal generator, the morphological stains may be contacted with the biological sample after the probing, inactivating and reprobing steps. For example, morphological stains like H&E may be sequentially imaged and registered after the methods disclosed herein.

In some embodiments, chromophores, fluorophores, enzymes, or enzyme substrates may be used as morphological stains. Suitable examples of chromophores that may be used as morphological stains (and their target cells, subcellular compartments, or cellular components) may include, but are not limited to, Eosin (alkaline cellular components, cytoplasm), Hematoxylin (nucleic acids), Orange G (red blood, pancreas, and pituitary cells), Light Green SF (collagen), Romanowsky-Giemsa (overall cell morphology), May-Grunwald (blood cells), Blue Counterstain (Trevigen), Ethyl Green (CAS) (amyloid), Feulgen-Naphthol Yellow S (DNA), Giemsa (differentially stains various cellular compartments), Methyl Green (amyloid), pyronin (nucleic acids), Naphthol-Yellow (red blood cells), Neutral Red (nuclei), Papanicolaou stain (a mixture of Hematoxylin, Eosin Y, Orange G and Bismarck Brown mixture (overall cell morphology)), Red Counterstain B (Trevigen), Red Counterstain C (Trevigen), Sirius Red (amyloid), Feulgen reagent (pararosanilin) (DNA), Gallocyanin chrom-alum (DNA), Gallocyanin chrom-alum and Naphthol Yellow S (DNA), Methyl Green-Pyronin Y (DNA), Thionin-Feulgen reagent (DNA), Acridine Orange (DNA), Methylene Blue (RNA and DNA), Toluidine Blue (RNA and DNA), Alcian blue (carbohydrates), Ruthenium Red (carbohydrates), Sudan Black (lipids), Sudan IV (lipids), Oil Red-O (lipids), Van Gieson's trichrome stain (acid fuchsin and picric acid mixture) (muscle cells), Masson trichrome stain (hematoxylin, acid fuchsin, and Light Green mixture) (stains collagen, cytoplasm, nucleioli differently), Aldehyde Fuchsin (elastin fibers), or Weigert stain (differentiates reticular and collagenous fibers).

Examples of suitable fluorescent morphological stains and if applicable, their target cells, subcellular compartments, or cellular components , may include, but are not limited to4',6-diamidino-2-phenylindole (DAPI) (nucleic acids), Eosin (alkaline cellular components, cytoplasm), Hoechst 33258 and Hoechst 33342 (two bisbenzimides) (nucleic acids), Propidium Iodide (nucleic acids), Spectrum Orange (nucleic acids), Spectrum Green (nucleic acids), Quinacrine (nucleic acids), Fluorescein-phalloidin (actin fibers), Chromomycin A 3 (nucleic acids), Acriflavine-Feulgen reaction (nucleic acid), Auramine O-Feulgen reaction (nucleic acids), Ethidium Bromide (nucleic acids). Nissl stains (neurons), high affinity DNA fluorophores such as POPO, BOBO, YOYO and TOTO and others, and Green Fluorescent Protein fused to DNA binding protein, such as histones, ACMA, Quinacrine and Acridine Orange.

Examples of suitable enzymes ,and their primary cellular locations or activities, may include, but are not limited to, ATPases (muscle fibers), succinate dehydrogenases (mitochondria), cytochrome c oxidases (mitochondria), phosphorylases (mitochondria), phosphofructokinases (mitochondria), acetyl cholinesterases (nerve cells), lactases (small intestine), acid phosphatases (lysosomes), leucine aminopeptidases (liver cells), dehydrogenases (mitochondria), myodenylate deaminases (muscle cells), NADH diaphorases (erythrocytes), and sucrases (small intestine).

Incertain embodiments, a morphological stain may be stable towards the inactivating agent, that is, the signal generating properties of the morphological stain may not be substantially affected by the inactivating agent. In some embodiments, where a biological sample may be stained with a probe and a morphological stain at the same time, application of inactivating agent to modify the signal from the probe may not modify the signal from the morphological stain. In some embodiments, a morphological stain may be used as a control to co-register the molecular information, obtained through the iterative probing steps, and the morphological information, obtained through the morphological stains.

The methods disclosed herein involving the detection of protein, RNA, and DNA generally involve the use of binders that physically bind to the target in a specific manner. As such, in some embodiments, a binder may bind to a target with sufficient specificity, that is, a binder may bind to a target with greater affinity than it does to any other molecule. In some embodiments, the binder may bind to other molecules, but the binding may be such that the non-specific binding may be at or near background levels. In some embodiments, the affinity of the binder for the target of interest may be in a range that is at least 2-fold, at least 5-fold, at least 10-fold, or more than its affinity for other molecules. In some embodiments, binders with the greatest differential affinity may be employed, although they may not be those with the greatest affinity for the target.

In some embodiments, binding between the target and the binder may be affected by physical binding. Physical binding may include binding effected using non-covalent interactions. Non-covalent interactions may include, but are not limited to, hydrophobic interactions, ionic interactions, hydrogen-bond interactions, or affinity interactions (such as, biotin-avidin or biotin-streptavidin complexation). In some embodiments, the target and the binder may have areas on their surfaces or in cavities giving rise to specific recognition between the two resulting in physical binding. In some embodiments, a binder may bind to a biological target based on the reciprocal fit of a portion of their molecular shapes.

Binders and their corresponding targets may be considered as binding pairs, of which non-limiting examples include immune-type binding-pairs, such as, antigen/antibody, antigen/antibody fragment, or hapten/anti-hapten; nonimmune-type binding-pairs, such as biotin/avidin, biotin/streptavidin, folic acid/folate binding protein, hormone/hormone receptor, lectin/specific carbohydrate, enzyme/enzyme, enzyme/substrate, enzyme/substrate analog, enzyme/pseudo-substrate (substrate analogs that cannot be catalyzed by the enzymatic activity), enzyme/co-factor, enzyme/modulator, enzyme/inhibitor, or vitamin B12/intrinsic factor. Other suitable examples of binding pairs may include complementary nucleic acid fragments (including DNA sequences, RNA sequences, LNA sequences, and PNA sequences); Protein A/antibody; Protein G/antibody; nucleic acid/nucleic acid binding protein; or polynucleotide/polynucleotide binding protein.

In some embodiments, the binder may be a sequence-or structure-specific binder, wherein the sequence or structure of a target recognized and bound by the binder may be sufficiently unique to that target.

In some embodiments, the binder may be structure-specific and may recognize a primary, secondary, or tertiary structure of a target. A primary structure of a target may include specification of its atomic composition and the chemical bonds connecting those atoms (including stereochemistry), for example, the type and nature of linear arrangement of amino acids in a protein. A secondary structure of a target may refer to the general three-dimensional form of segments of biomolecules, for example, for a protein a secondary structure may refer to the folding of the peptide "backbone" chain into various conformations that may result in distant amino acids being brought into proximity with each other. Suitable examples of secondary structures may include, but are not limited to, alpha helices, beta pleated sheets, or random coils. A tertiary structure of a target may be is its overall three dimensional structure. A quaternary structure of a target may be the structure formed by its noncovalent interaction with one or more other targets or macromolecules (such as protein interactions). An example of a quaternary structure may be the structure formed by the four-globin protein subunits to make hemoglobin. A binder in accordance with the embodiments of the invention may be specific for any of the afore-mentioned structures.

An example of a structure-specific binder may include a protein-specific molecule that may bind to a protein target. Examples of suitable protein-specific molecules may include antibodies and antibody fragments, nucleic acids (for example, aptamers that recognize protein targets), or protein substrates (non-catalyzable).

In some embodiments, a binder may be sequence-specific. A sequence-specific binder may include a nucleic acid and the binder may be capable of recognizing a particular linear arrangement of nucleotides or derivatives thereof in the target. In some embodiments, the linear arrangement may include contiguous nucleotides or derivatives thereof that may each bind to a corresponding complementary nucleotide in the binder. In an alternate embodiment, the sequence may not be contiguous as there may be one, two, or more nucleotides that may not have corresponding complementary residues on the probe. Suitable examples of nucleic acid-based binders may include, but are not limited to, DNA or RNA oligonucleotides or polynucleotides. In some embodiments, suitable nucleic acids may include nucleic acid analogs, such as dioxygenin dCTP, biotin dcTP 7-azaguanosine, azidothymidine, inosine, or uridine.

Regardless of the type of binder and the target, in protein, DNA, and RNA detection, the specificity of binding between the binder and the target may also be affected depending on the binding conditions (for example, hybridization conditions in case of complementary nucleic acids). Suitable binding conditions may be realized by modulation one or more of pH, temperature, or salt concentration.

A binder may be intrinsically labeled (signal generator or enzyme attached during synthesis of binder) or extrinsically labeled (signal generator or enzyme attached during a later step). For example for a protein-based binder, an intrinsically labeled binder may be prepared by employing labeled amino acids. Similarly, an intrinsically labeled nucleic acid may be synthesized using methods that incorporate signal generator-labeled nucleotides directly into the growing nucleic acid. In some embodiments, a binder may be synthesized in a manner such that signal generators or enzymes may be incorporated at a later stage. For example, this latter labeling may be accomplished by chemical means by the introduction of active amino or thiol groups into nucleic acids of peptide chains. In some embodiments, a binder such a protein (for example, an antibody) or a nucleic acid (for example, a DNA) may be directly chemically labeled using appropriate chemistries.

In some embodiments, combinations of binders may be used that may provide greater specificity or in certain embodiments amplification of the signal. Thus, in some embodiments, a sandwich of binders may be used, where the first binder may bind to the target and serve to provide for secondary binding, where the secondary binder may or may not include a label, which may further provide for tertiary binding (if required) where the tertiary binding member may include a label.

Suitable examples of binder combinations may include primary antibody-secondary antibody, complementary nucleic acids, or other ligand-receptor pairs (such as biotin-streptavidin). Some specific examples of suitable binder pairs may include mouse anti-myc for recombinant expressed proteins with c-myc epitope; mouse anti-HisG for recombinant protein with His-Tag epitope, mouse anti-xpress for recombinant protein with epitope- tag, rabbit anti-goat for goat IgG primary molecules, complementary nucleic acid sequence for a nucleic acid; mouse anti-thio for thioredoxin fusion proteins, rabbit anti-GFP for fusion protein, jacalin for α-D-galactose; and melibiose for carbohydrate-binding proteins, sugars, nickel couple matrix or heparin.

In some embodiments, a combination of a primary antibody and a secondary antibody may be used as a binder. A primary antibody may be capable of binding to a specific region of the target and the secondary antibody may be capable of binding to the primary antibody. A secondary antibody may be attached to a signal generator or an enzyme before binding to the primary antibody or may be capable of binding to a signal generator or an enzyme at a later step. In an alternate embodiment, a primary antibody and specific binding ligand-receptor pairs (such as biotin-streptavidin) may be used. The primary antibody may be attached to one member of the pair (for example biotin) and the other member (for example streptavidin) may be labeled with a signal generator or an enzyme. The secondary antibody, avidin, streptavidin, or biotin may be each independently labeled with a signal generator or an enzyme.

In some embodiments, the methods disclosed herein may be employed in an immunostaining procedure, and a primary antibody may be used to specifically bind a target protein. A secondary antibody may be used to specifically bind to the primary antibody, thereby forming a bridge between the primary antibody and a subsequent reagent (for example a signal generator or enzyme), if any. For example, a primary antibody may be mouse IgG (an antibody created in mouse) and the corresponding secondary antibody may be goat anti-mouse (antibody created in goat) having regions capable of binding to a region in mouse IgG.

In some embodiments, signal amplification may be obtained when several secondary antibodies may bind to epitopes on the primary antibody. In an immunostaining procedure a primary antibody may be the first antibody used in the procedure and the secondary antibody may be the second antibody used in the procedure. In some embodiments, a primary antibody may be the only antibody used in an immunostaining procedure.

The type of signal generator suitable for the methods disclosed herein may depend on a variety of factors, including the nature of the analysis being conducted, the type of the energy source and detector used, the type of inactivating agent employed, the type of binder, the type of target, or the mode of attachment between the binder and the signal generator (e.g., cleavable or non-cleavable).

A suitable signal generator may include a molecule or a compound capable of providing a detectable signal. A signal generator may provide a characteristic signal following interaction with an energy source or a current. An energy source may include electromagnetic radiation source and a fluorescence excitation source. Electromagnetic radiation source may be capable of providing electromagnetic energy of any wavelength including visible, infrared and ultraviolet. Electromagnetic radiation may be in the form of a direct light source or may be emitted by a light emissive compound such as a donor fluorophore. A fluorescence excitation source may be capable of making a source fluoresce or may give rise to photonic emissions (that is, electromagnetic radiation, directed electric field, temperature, physical contact, or mechanical disruption). Suitable signal generators may provide a signal capable of being detected by a variety of methods including optical measurements (for example, fluorescence), electrical conductivity, or radioactivity. Suitable signal generators may be, for example, light emitting, energy accepting, fluorescing, radioactive, or quenching.

A suitable signal generator may be sterically and chemically compatible with the constituents to which it is bound, for example, a binder. Additionally, a suitable signal generator may not interfere with the binding of the binder to the target, nor may it affect the binding specificity of the binder. A suitable signal generator may be organic or inorganic in nature. In some embodiments, a signal generator may be of a chemical, peptide or nucleic acid nature.

A suitable signal generator may be directly detectable. A directly detectable moiety may be one that may be detected directly by its ability to emit a signal, such as for example a fluorescent label that emits light of a particular wavelength following excitation by light of another lower, characteristic wavelength and/or absorb light of a particular wavelength.

A signal generator, suitable in accordance with the methods disclosed herein may be amenable to manipulation on application of a chemical agent. In some embodiments, a signal generator may be capable of being chemically destroyed on exposure to an inactivating agent. Chemical destruction may include complete disintegration of the signal generator or modification of the signal-generating component of the signal generator. Modification of the signal-generating component may include any chemical modification (such as addition, substitution, or removal) that may result in the modification of the signal generating properties. For example, unconjugating a conjugated signal generator may result in destruction of chromogenic properties of the signal generator. Similarly, substitution of a fluorescence-inhibiting functional group on a fluorescent signal generator may result in modification of its fluorescent properties. In some embodiments, one or more signal generators substantially resistant to inactivation by a specific chemical agent may be used as a control probe in the provided methods.

In some embodiments, a signal generator may be selected from a light emissive molecule, a radioisotope (e.g., P³² or H³, ¹⁴C, ¹²⁵I and ¹³¹I), an optical or electron density marker, a Raman-active tag, an electron spin resonance molecule (such as for example nitroxyl radicals), an electrical charge transferring molecule (i.e., an electrical charge transducing molecule), a semiconductor nanocrystal, a semiconductor nanoparticle, a colloid gold nanocrystal, a microbead, a magnetic bead, a paramagnetic particle, or a quantum dot.

In some embodiments, a signal generator may include a light-emissive molecule. A light emissive molecule may emit light in response to irradiation with light of a particular wavelength. Light emissive molecules may be capable of absorbing and emitting light through luminescence (non-thermal emission of electromagnetic radiation by a material upon excitation), phosphorescence (delayed luminescence as a result of the absorption of radiation), chemiluminescence (luminescence due to a chemical reaction), fluorescence, or polarized fluorescence.

In some embodiments, a signal generator may essentially include a fluorophore. In some embodiments, a signal generator may essentially include a fluorophore attached to an antibody, for example, in an immunohistochemistry analysis. Suitable fluorophores that may be conjugated to a primary antibody include, but are not limited to, Fluorescein, Rhodamine, Texas Red, VECTOR Red, ELF (Enzyme-Labeled Fluorescence), Cy2, Cy3, Cy3.5, Cy5, Cy7, FluorX, Calcein, Calcein-AM, CRYPTOFLUOR, Orange (42 kDa), Tangerine (35 kDa), Gold (31 kDa), Red (42 kDa), Crimson (40 kDa), BHMP, BHDMAP, Br-Oregon, Lucifer Yellow, Alexa dye family, N-[6-(7-nitrobenz-2-oxa-1,3-diazol-4-yl)amino]caproyl] (NBD), BODIPY, boron dipyrromethene difluoride, Oregon Green, MITOTRACKER Red, Phycoerythrin, Phycobiliproteins BPE (240 kDa) RPE (240 kDa) CPC (264 kDa) APC (104 kDa), Spectrum Blue, Spectrum Aqua, Spectrum Green, Spectrum Gold, Spectrum Orange, Spectrum Red, Infra-Red (IR) Dyes, Cyclic GDP-Ribose (cGDPR), Calcofluor White, Lissamine, Umbelliferone, Tyrosine or Tryptophan. In some embodiments, a signal generator may essentially include a cyanine dye. In some embodiments, a signal generator may essentially include one or more a Cy3 dye, a Cy5 dye, or a Cy7 dye.

In some embodiments, the signal generator may be part of a FRET pair. FRET pair includes two fluorophores that are capable of undergoing FRET to produce or eliminate a detectable signal when positioned in proximity to one another. Some examples of donors may include Alexa 488, Alexa 546, BODIPY 493, Oyster 556, Fluor (FAM), Cy3, or TTR (Tamra). Some examples of acceptors may include Cy5, Alexa 594, Alexa 647, or Oyster 656.

As described hereinabove, one or more of the aforementioned molecules may be used as a signal generator. In some embodiments, one or more of the signal generators may not be amenable to chemical destruction and a cleavable linker may be employed to associate the signal generator and the binder. In some embodiments, one or more of the signal generators may be amenable to signal destruction and the signal generator may essentially include a molecule capable of being destroyed chemically. In some embodiments, a signal generator may include a fluorophore capable of being destroyed chemically by an oxidizing agent. In some embodiments, a signal generator may essentially include cyanine, coumarin, BODIPY, ATTO 658, a quantum dot or ATTO 634, capable of being destroyed chemically by an oxidizing agent. In some embodiments, a signal generator may include one or more a Cy3 dye, a Cy5 dye, or a Cy7 dye capable of being destroyed or quenched.

In some embodiments, a probe may include a binder coupled to an enzyme. In some embodiments, a suitable enzyme catalyzes a chemical reaction of the substrate to form a reaction product that can bind to a receptor (e.g., phenolic groups) present in the sample or a solid support to which the sample is bound. A receptor may be exogeneous (that is, a receptor extrinsically adhered to the sample or the solid-support) or endogeneous (receptors present intrinsically in the sample or the solid-support). Signal amplification may be effected as a single enzyme may catalyze a chemical reaction of the substrate to covalently bind multiple signal generators near the target.

In some embodiments, a suitable enzyme may also be capable of being inactivated by an oxidizing agent. Examples of suitable enzymes include peroxidases, oxidases, phosphatases, esterases, and glycosidases. Specific examples of suitable enzymes include horseradish peroxidase, alkaline phosphatase, β-D-galactosidase, lipase, and glucose oxidase. In some embodiments, the enzyme is a peroxidase selected from horseradish peroxidase, cytochrome C peroxidase, glutathione peroxidase, microperoxidase, myeloperoxidase, lactoperoxidase, and soybean peroxidase.

In some embodiments, a binder and an enzyme may be embodied in a single entity, for example a protein molecule capable of binding to a target and also catalyzing a chemical reaction of substrate. In other embodiments, a binder and an enzyme may be embodied in separate entities and may be coupled by covalent bond formation or by using ligand-receptor conjugate pairs (e.g., biotin streptavidin).

An enzyme substrate may be selected depending on the enzyme employed and the target available for binding in the sample or on the solid support. For example, in embodiments including HRP as an enzyme, a substrate may include a substituted phenol (e.g., tyramine). Reaction of HRP to the tyramine may produce an activated phenolic substrate that may bind to endogeneous receptors like electron-rich moieties (such as tyrosine or tryptophan) or phenolic groups present in the surface proteins of a biological sample. In alternate embodiments, where 3-methyl-2-benzothiazolinone hydrochloride (MBTH) may be employed as a substrate along with an HRP enzyme, exogeneous receptors like p-dimethylaminobenzaldehyde (DMAB) may be adhered to the solid support or the biological sample before reacting with the substrate.

In some embodiments, an enzyme substrate may be dephosphorylated after reaction with the enzyme. The dephosphorylated reaction product may be capable of binding to endogeneous or exogeneous receptors (e.g., antibodies) in the sample or the solid-support. For example, an enzyme may include alkaline phosphatase (AP) and a substrate may include NADP, substituted phosphates (e.g., nitrophenyl phosphate), or phosphorylated biotin. The receptors may include NAD binding proteins, antibodies to the dephosphorylated reaction product (e.g., anti nitro-phenol), avidin, or streptavidin accordingly.

In some embodiments, an enzyme may include β-galactosidase and a substrate may include β-galactopryanosyl-glycoside of fluorescein or coumarin. Receptors may include antibodies to deglycosylated moieties (e.g., anti-fluorescein or anti-coumarin). In some embodiments, multiple enzyme combinations like HRP/AP may be used as an enzyme. A substrate may include phosphorylated substituted phenol e.g., tyrosine phosphate, which may be dephosphorylated by AP before reacting with HRP to form a reaction product capable of binding to phenolic groups or electron rich moieties-based receptors.

A reaction product of the enzyme substrate may further be capable of being providing a detectable signal. In some embodiments, enzyme substrates employed in the methods disclosed herein may include non-chromogenic or non-chemiluminescent substrates, that is a reaction of the enzyme and the enzyme substrate may not itself produce a detectable signal. Enzyme substrates employed in the methods disclosed herein may include an extrinsic signal generator (e.g., a fluorophore) as a label. The signal generator and the enzyme substrate may be attached directly (e.g., an enzyme substrate with a fluorescent label) or indirectly (e.g., through ligand-receptor conjugate pair). In some embodiments, a substrate may include protected functional groups (e.g., sulfhydryl groups). After binding of the activated substrate to the receptors, the functional group may be deprotected and conjugation to a signal generator effected using a signal generator having a thiol reactive group (e.g., maleimide or iodoacetyl).

In some embodiments, a label may include horseradish peroxidase and the substrate is selected from substituted phenols (e.g., tyramine). In some embodiments, the horseradish peroxidase causes the activated phenolic substrate to covalently bind to phenolic groups present in the sample or a solid support to which the sample is bound. In some embodiments, a probe may include a binder coupled to HRP and a substrate may include tyramine-coupled to a fluorophore.

A chemical agent may include one or chemicals capable of modifying the signal generator, the enzyme, or the cleavable linker (if present) between the signal generator and the binder or the enzyme substrate. A chemical agent may be contacted with the sample in the form of a solid, a solution, a gel, or a suspension.

In some embodiments, a chemical agent may include oxidizing agents, for example, active oxygen species, hydroxyl radicals, singlet oxygen, hydrogen peroxide, or ozone. In some embodiments, a chemical agent may include hydrogen peroxide, potassium permanganate, sodium dichromate, aqueous bromine, iodine-potassium iodide, or t-butyl hydroperoxide

One or more of the aforementioned chemical agents may be used in the methods disclosed herein depending upon the susceptibility of the signal generator, of the enzyme, of the binder, of the target, or of the biological sample to the chemical agent. In some embodiments, a chemical agent that essentially does not affect the integrity of the binder, the target, and the biological sample may be employed. In some embodiments, a chemical agent that does not affect the specificity of binding between the binder and the target may be employed. Refering to steps B,F, and J wherein the specific RNA, protein, or DNA targets are detected or osbserved, in some embodiments, the steps may include a quantitative measurement of at least one target in the sample. In some embodiments, an intensity value of a signal (for example, fluorescence intensity) may be measured and may be correlated to the amount of target in the biological sample. A correlation between the amount of target and the signal intensity may be determined using calibration standards. In some embodiment, intensity values of the first and second signals may be measured and correlated to the respective target amounts. In some embodiments, by comparing the two signal intensities, the relative amounts of the first target and the second target (with respect to each other or with respect to a control) may be ascertained. Similarly, where multiple targets may be analyzed using multiple probes, relative amounts of different targets in the biological sample may be determined by measuring different signal intensities. In some embodiments, one or more control samples may be used as described hereinabove. By observing a presence or absence of a signal in the samples (biological sample of interest versus a control), information regarding the biological sample may be obtained. For example by comparing a diseased tissue sample versus a normal tissue sample, information regarding the targets present in the diseased tissue sample may be obtained. Similarly by comparing signal intensities between the samples (i.e., sample of interest and one or more control), information regarding the expression of targets in the sample may be obtained.

In some embodiments, the detecting steps include co-localizing at least two targets in the sample. Methods for co-localizing targets in a sample are described in U.S. Patent Application Serial No. 11/686,649, entitled "System and Methods for Analyzing Images of Tissue Samples", filed on March 15, 2007; U.S. Patent Application Serial No. 11/500028, entitled "System and Method for Co-Registering Multi-Channel Images of a Tissue Micro Array", filed on August 7, 2006; U.S. Patent Application Serial No. 11/606582, entitled "System and Methods for Scoring Images of a Tissue Micro Array, filed on November 30, 2006, and U.S. Patent. Application Serial No. 11/680063, entitled Automated Segmentation of Image Structures, filed on February 28, 2007.

In some embodiments, a location of the signal in the biological sample may be observed. In some embodiments, a localization of the signal in the biological signal may be observed using morphological stains. In some embodiments relative locations of two or more signals may be observed. A location of the signal may be correlated to a location of the target in the biological sample, providing information regarding localization of different targets in the biological sample. In some embodiments, an intensity value of the signal and a location of the signal may be correlated to obtain information regarding localization of different targets in the biological sample. For examples certain targets may be expressed more in the cytoplasm relative to the nucleus, or vice versa. In some embodiments, information regarding relative localization of targets may be obtained by comparing location and intensity values of two or more signals.

In some embodiments, one or more of the observing or correlating step may be performed using computer-aided means. In embodiments where the signal(s) from the signal generator may be stored in the form of digital image(s), computer-aided analysis of the image(s) may be conducted. In some embodiments, images (e.g., signals from the probe(s) and morphological stains) may be overlaid using computer-aided superimposition to obtain complete information of the biological sample, for example topological and correlation information.

In some embodiments, one or more of the aforementioned process steps may be automated and may be performed using automated systems. In some embodiments, all the steps may be performed using automated systems.

The methods disclosed herein may find applications in analytic, diagnostic, and therapeutic applications in biology and in medicine. In some embodiments, the methods disclosed herein may find applications in histochemistry, particularly, immunohistochemistry. Analysis of cell or tissue samples from a patient, according to the methods described herein, may be employed diagnostically (e.g., to identify patients who have a particular disease, have been exposed to a particular toxin or are responding well to a particular therapeutic or organ transplant) and prognostically (e.g., to identify patients who are likely to develop a particular disease, respond well to a particular therapeutic or be accepting of a particular organ transplant). The methods disclosed herein, may facilitate accurate and reliable analysis of a plurality (e.g., potentially infinite number) of targets (e.g., disease markers) from the same biological sample.

### EXPERIMENTAL

### Preparation of Tissue Samples

Human lung tissue samples were obtained as tissue slides embedded in paraffin. The samples included one microarray of normal, premalignant, and cancer tissues with progressive grades (Pantomics, LUC961) and four whole tissue lung cancer samples (Wood Hudson Cancer Research Center).

The paraffin embedded slides were baked at 60°C for one hour with tissue facing up and parallel to the oven rack. After baking, slides were deparaffinized by washing in xylene with gentle agitation for ten minutes. The samples were then rehydrated by washing in four solutions of ethanol with concentrations decreasing in the order of 100%, 95%, 70%, and 50% followed by a wash with 1x phosphate buffer saline (PBS, pH 7.4). After rehydration, the slides were washed with 1x PBS. A ten minute wash in 0.3% Triton X-100 in PBS was performed for membrane permeabilization of the tissue, followed by a wash with 1x PBS.

### U6 snRNA staining

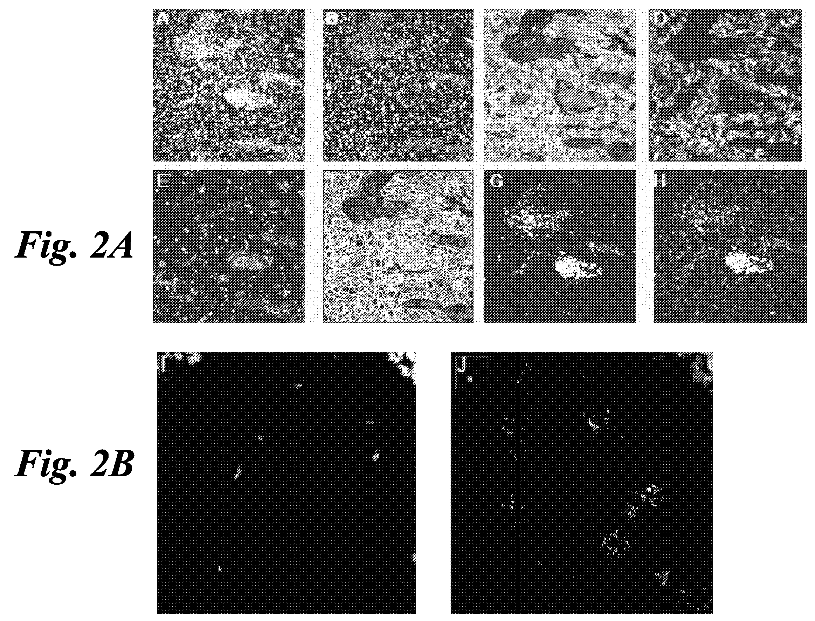

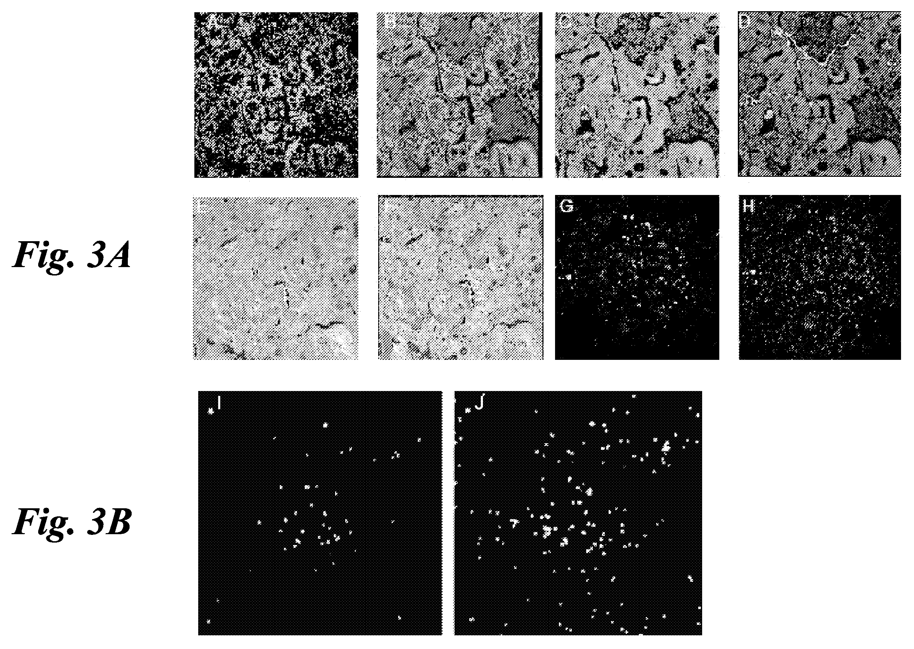

After permeabilization, slides were incubated with prehybridization buffer (1x Exiqon buffer, Exiqon, miRCURY LNA™ microRNA ISH Optimization Kit) for one hour at room temperature then hybridized with 100ul of 50mM TYE665 labeled U6 probe (custom U6 probe from Exiqon, Inc.) in Exiqon buffer at 50°C overnight in Thermobrite. Slides were washed with 0.5xSSC and 0.2x SSC at 50°C for 10min then rinsed with 0.1x SSC briefly. Slides were DAPI stained at room temperature for 15min and mounted with a mounting medium. The images were taken on Olympus microscope with a 20x objective. The images are shown in FIGs. 2 and 3 panel A: nuclei stained with DAPI, panel B: U6 snRNA stained with TYE665.

### Antigen Retrieval

After the RNA detection process, slides were treated with dual-buffer heat-induced epitope retrieval. Using a pressure cooker the slides were exposed to Citrate Buffer pH 6.0 (Vector Unmasking Solution), under pressure for twenty minutes and then transferred to hot Tris-EDTA Buffer pH 9.0 and allowed to stand in the cooker at atmospheric pressure for twenty minutes. This was followed by cooling down at room temperature for ten minutes and a series of washes in 1xPBS.

### Blocking

Following antigen retrieval the slides were blocked against nonspecific binding by incubating overnight in a 10% donkey serum, 3% bovine serum albumin (BSA) solution at 4°C.

### Protein Staining

Slides were stained with DAPI and coverslipped. Images were taken at 20x prior to protein staining to baseline the autofluorescence from Cy3 and Cy5 channels, using same fields of view as those used for RNA detection. Slides were decoverslipped in 1xPBS and stained with a cocktail of Cy3-directly conjugated Cytokeratin-7 (Dako M7018, 20µg/mL) and Cy5-directly conjugated EGFR (Cell Signaling 4267, 20µg/mL) diluted in 3% BSA in 1X PBS. Incubation was for one hour at room temperature. After incubation, a series of washes in 1xPBS removed excess antibodies and slides were coverslipped. The samples were imaged (FIGs. 2 and 3, panel C: EGFR stained with Cy5 and panel D: Cytokeratin 7 stained with Cy3) and then treated with a basic H₂O₂ solution for 15 minutes to bleach the directly conjugated dyes. After 15 minutes, slides were washed with PBS and coverslipped. Images were taken again to baseline the autofluorescence after bleaching. Slides were stained with a second cocktail of Cy3-directly conjugated NaKATPase (Epitomics 2047, 5µg/mL) and Cy5-conjugated IGF1R (Lifespan Biosciences LS-C82136, 20µg/mL), coverslipped, and imaged again (FIGs. 2 and 3, panel E: IGF1R stained with Cy5 and panel F: NaKATPase stained with Cy3). Samples were bleached a second time before FISH processing.

### DNA FISH

To allow subsequent FISH staining coverslip was removed by incubation in 2xSSC buffer and slide was subjected to 10 min treatment with 0.05% pepsin that partially removed protein structures to allow access to nuclear DNA. Slide was then fixed using aqueous 4% formaldehyde solution for 10 min, washed and subjected to hybridization using FISH probes for EGFR (PlatinumBright415, aqua fluorophore), cMet (PlatinumBright550, red fluorophore) and Chromosome 7 centromere (PlatinumBright495, green fluorophore) and counterstained with DAPI. The hybridization was carried out by dehydrating the slide by passage through series of aqueous solutions of increasing concentration of ethanol followed by 100% ethanol and then allowed to dry briefly. The probe mixture was applied on the region of the slide containing tissue section, then covered with a coverslip and placed in a slide incubator capable of heating and cooling the slide. The slide containing the probe mixture was heated to 80°C for 10 min to denature DNA hybrids and allowed to cool to 37°C. The slide was then kept at that temperature for 16 hours. Slide was then washed in 2xSSC buffer containing 0.3% of detergent NP-40 and washed 2 min in 0.4xSSC containing 0.3% NP-40 at 72°C followed by counterstaining with DAPI. Next, the regions of tissue section that had invasive tumor were imaged using coordinates recorded in the immunofluorescence step. Image sets were recorded at 40x using filtersets specific to blue, green and red fluorophores and DAPI (FIGs. 2 and 3, panel G: cMET stained with PlatinumBright550 and panel H: EGFR gene stained with PlatinumBright415).

The foregoing embodiments are therefore to be considered in all respects as illustrative rather than limiting on the invention described herein. The scope of the invention is thus indicated by the appended claims rather than by the foregoing description, and all changes that come within the meaning and range of equivalency of the claims are therefore intended to be embraced therein.

## Claims

1. A method of probing multiple targets in a biological sample comprising the steps of:
(a) subjecting the sample to an in situ hybridization reaction using a labeled nucleic acid probe that directly or indirectly binds an RNA target;
(b) observing a signal from the labeled probe bound to the RNA target;
(c) optionally removing the signal from the labeled probe;
(d) subjecting the sample to an antigen retrieval protocol to retrieve the sample's protein epitopes;
(e) subjecting the sample to an in situ hybridization reaction using an antibody-based method and attaching one or more antibody probe to antigens on the sample;
(f) observing a signal from the one or more antibody probes;
(g) removing the signal from the antibody probes;
(h) optionally applying a protease treatment to access the sample's DNA targets;
(i) subjecting the sample to an in situ hybridization reaction using a labeled nucleic acid probe to directly or indirectly label one or more of the sample's DNA targets ;
(j) observing a signal from the labeled DNA targets;
(k) optionally removing the signal from the one or more labeled DNA targets.

2. The method of claim 1 wherein step a further comprises a prehybridization step to block DNA, the use of a blocking agent in the hybridization reaction, or a combination thereof.

3. The method of claim 1 wherein after observing a signal from the labeled probe bound to the RNA target the sample is subjected to step c, and steps a, b, and c are repeated one or more times with other labeled nucleic acid probes for different RNA targets.

4. The method of claim 1 wherein an *in situ* hybridization reaction using an antibody-based method comprises immunohistochemistry (IHC) or immunofluorescence (IF) techniques.

5. The method of claim 4 wherein antibody probe comprises a mixture of more than one probe.

6. The method of claim 4 wherein after observing and removing a signal from the one or more antibody probes, steps e, f, and g are repeated one or more times with another antibody probe for a different antigen.

7. The method of claim 1 further comprising after protease treatment, the step of preserving tissue morphology, prehybirdization , or a combination thereof.

8. The method of claim 1 wherein after observing a signal from the labeled probe bound to the DNA target, steps i, j, and k are repeated one or more times with another labeled nucleic acid probe for a different DNA target.

9. The method of claim 1 wherein the removing the signals in steps c, g, and i comprises a signal inactivation agent, photoreaction, photactivated chemical bleaching, probe stripping, oxidation, electron transfer, or a combination thereof.

10. The method of claim 1 further comprising staining the sample with one or more control probes to allow for registration of multiple images of the sample.

11. The method of claim 10 wherein the control probe is a morphological stain.

12. The method of claim 10 further comprising registering multiple images of the sample wherein registering comprises:
obtaining multiple images of the samples from steps b, f, j, or a combination thereof;
aligning and overlaying the multiple images according the the signals detected from the control probe.

13. The method of claim 12 further comprising the step of analyzing the expression of the protein, RNA, and DNA from the overlaid images.

14. The method of claim 1, further comprising measuring one or more intensity values of the signal observed in observing steps b, f, j, or a combination thereof.

15. The method of claim 14 wherein measuring one or more intensity values of the signal comprises a signal amplification technique.

## Patentansprüche

1. Verfahren zum Sondieren mehrerer Ziele in einer biologischen Probe, umfassend die Schritte:
(a) Unterziehen der Probe einer In-situ-Hybridisierungsreaktion unter Verwendung einer markierten Nucleinsäure-Sonde, welche direkt oder indirekt an ein RNA-Ziel bindet;
(b) Beobachten eines Signals von der markierten Sonde, welche an das RNA-Ziel gebunden ist;
(c) optional Entfernen des Signals von der markierten Sonde;
(d) Unterziehen der Probe einem Antigenrückgewinnungsprotokoll, um die Protein-Epitope der Probe zurückzugewinnen;
(e) Unterziehen der Probe einer In-situ-Hybridisierungsreaktion unter Verwendung eines antikörperbasierten Verfahrens und Anhängen einer oder mehrerer Antikörpersonde(n) an Antigene auf der Probe;
(f) Beobachten eines Signals von der einen oder den mehreren Antikörpersonde(n);
(g) Entfernen des Signals von den Antikörpersonden;
(h) optional Anwenden einer Protease-Behandlung, um auf die DNA-Ziele der Probe zuzugreifen;
(i) Unterziehen der Probe einer In-situ-Hybridisierungsreaktion unter Verwendung einer markierten Nucleinsäure-Sonde, um direkt oder indirekt ein oder mehrere DNA-Ziel(e) der Probe zu markieren;
(j) Beobachten eines Signals von den markierten DNA-Zielen;
(k) optional Entfernen des Signals von dem einen oder den mehreren markierten DNA Ziel(en).

2. Verfahren nach Anspruch 1, wobei Schritt a des Weiteren einen Prähybridisierungsschritt zur Blockierung von DNA, die Verwendung eines Blockierungsmittels in der Hybridisierung oder eine Kombination davon umfasst.

3. Verfahren nach Anspruch 1, wobei nach Beobachten eines Signals von der markierten Sonde, die an das RNA-Ziel gebunden ist, die Probe Schritt c unterzogen wird und Schritte a, b und c einmal oder mehrere Male mit anderen markierten Nucleinsäure-Sonden für verschiedene RNA-Ziele wiederholt werden.

4. Verfahren nach Anspruch 1, wobei eine In-situ-Hybridisierungsreaktion, die ein antikörperbasiertes Verfahren verwendet, Immunhistochemie- (IHC) oder Immunfluoreszenz- (IF) Techniken umfasst.

5. Verfahren nach Anspruch 4, wobei die Antikörpersonde ein Gemisch von mehr als einer Sonde enthält.

6. Verfahren nach Anspruch 4, wobei nach Beobachten und Entfernen eines Signals von der einen oder den mehreren Antikörpersonde(n), die Schritte e, f und g einmal oder mehrere Male mit einer anderen Antikörpersonde für ein anderes Antigen wiederholt werden.

7. Verfahren nach Anspruch 1, des Weiteren umfassend, nach der Protease-Behandlung, den Schritt zum Konservieren von Gewebemorphologie, Prähybridisieren oder eine Kombination davon.

8. Verfahren nach Anspruch 1, wobei nach Beobachten eines Signals von der markierten Sonde, die an das DNA-Ziel gebunden ist, die Schritte i, j und k einmal oder mehrere Male mit einer anderen markierten Nucleinsäure-Sonde für ein anderes DNA-Ziel wiederholt werden.

9. Verfahren nach Anspruch 1, wobei das Entfernen der Signale in den Schritten c, g und i einen Signalinaktivierungswirkstoff, eine Photoreaktion, ein photoaktiviertes chemisches Bleichen, ein Sondenstripping, eine Oxidation, einen Elektronentransfer oder eine Kombination davon umfasst.

10. Verfahren nach Anspruch 1, des Weiteren umfassend ein Färben der Probe mit einer oder mehreren Kontrollsonde(n), um eine Registrierung mehrerer Bilder der Probe zu ermöglichen.

11. Verfahren nach Anspruch 10, wobei die Kontrollsonde eine morphologische Färbung ist.

12. Verfahren nach Anspruch 10, des Weiteren umfassend ein Registrieren mehrerer Bilder der Probe, wobei das Registrieren umfasst:
Erhalten mehrerer Bilder der Proben aus den Schritten b, f, j oder einer Kombination davon;
Ausrichten und Überlagern der mehreren Bilder gemäß den Signalen, welche von der Kontrollsonde erfasst sind.

13. Verfahren nach Anspruch 12, des Weiteren umfassend den Schritt zum Analysieren der Expression des Proteins, der RNA und DNA aus den überlagerten Bildern.

14. Verfahren nach Anspruch 1, des Weiteren umfassend ein Messen eines Intensitätswerts oder mehrerer Intensitätswerte des Signals, welches in den Beobachtungsschritten b, f, j oder einer Kombination davon beobachtet werden.

15. Verfahren nach Anspruch 14, wobei das Messen eines Intensitätswerts oder mehrerer Intensitätswerte des Signals eine Signalamplifizierungstechnik umfasst.

## Revendications

1. Procédé de détection de cibles multiples dans un échantillon biologique comprenant les étapes consistant à :
a) soumettre l'échantillon à une réaction d'hybridation in situ en utilisant une sonde d'acide nucléique marquée qui se lie directement ou indirectement à une cible d'ARN ;
b) observer un signal provenant de la sonde marquée liée à la cible d'ARN ;
c) éliminer éventuellement le signal de la sonde marquée ;
d) soumettre l'échantillon à un protocole de récupération d'antigène pour récupérer les épitopes de protéine de l'échantillon ;
e) soumettre l'échantillon à une réaction d'hybridation in situ en utilisant un procédé à base d'anticorps et en fixant une sonde ou plusieurs sondes d'anticorps à des antigènes sur l'échantillon ;
f) observer un signal provenant des une ou plusieurs sondes d'anticorps ;
g) éliminer le signal provenant des sondes d'anticorps ;
h) appliquer éventuellement un traitement à la protéase pour accéder aux cibles d'ADN de l'échantillon ;
i) soumettre l'échantillon à une réaction d'hybridation in situ en utilisant une sonde d'acide nucléique marquée afin de marquer de manière directe ou indirecte une ou plusieurs cibles d'ADN de l'échantillon ;
j) observer un signal provenant des cibles d'ADN marquées ;
k) éliminer éventuellement le signal des un ou plusieurs cibles d'ADN marquées.

2. Procédé selon la revendication 1, dans lequel l'étape a comprend en outre une étape de pré-hybridation pour bloquer l'ADN, l'utilisation d'un agent bloquant dans la réaction d'hybridation ou une combinaison des deux.

3. Procédé selon la revendication 1, dans lequel, après observation d'un signal provenant de la sonde marquée liée à la cible d'ARN, l'échantillon est soumis à l'étape c et les étapes a, b et c sont répétées une ou plusieurs fois avec d'autres sondes d'acide nucléique marquées pour différentes cibles d'ARN.

4. Procédé selon la revendication 1, dans lequel une réaction d'hybridation in situ utilisant un procédé à base d'anticorps comprend des techniques d'immunohistochimie (IHC) ou d'immunofluorescence (IF).

5. Procédé selon la revendication 4, dans lequel la sonde d'anticorps comprend un mélange de plus d'une sonde.

6. Procédé selon la revendication 4, dans lequel, après observation et élimination d'un signal provenant des une ou plusieurs sondes d'anticorps, les étapes e, f et g sont répétées une ou plusieurs fois avec une autre sonde d'anticorps pour un antigène différent.

7. Procédé selon la revendication 1, comprenant en outre, après traitement à la protéase, l'étape de conservation de la morphologie du tissu, de la pré-hybridation ou d'une de leurs combinaisons.

8. Procédé selon la revendication 1, dans lequel, après observation d'un signal provenant de la sonde marquée liée à la cible d'ADN, les étapes i, j et k sont répétées une ou plusieurs fois avec une autre sonde d'acide nucléique marquée pour une cible d'ADN différente.

9. Procédé selon la revendication 1, dans lequel l'élimination des signaux aux étapes c, g et i comprend un agent d'inactivation de signal, une photoréaction, un blanchiment chimique photo-activé, une extraction de sonde, une oxydation, un transfert d'électrons ou une de leurs combinaisons.

10. Procédé selon la revendication 1, comprenant en outre la coloration de l'échantillon par une ou plusieurs sondes témoins pour permettre l'enregistrement de multiples images de l'échantillon.

11. Procédé selon la revendication 10, dans lequel la sonde témoin est une coloration morphologique.

12. Procédé selon la revendication 10, comprenant en outre l'enregistrement de multiples images, dans lequel l'enregistrement comprend :
l'obtention de multiples images des échantillons des étapes b, f, j ou d'une de leurs combinaisons ;
l'alignement et la superposition des images multiples selon les signaux détectés en provenance de la sonde témoin.

13. Procédé selon la revendication 12, comprenant en outre l'étape d'analyse de l'expression de la protéine, de l'ARN et de l'ADN à partir des images superposées.

14. Procédé selon la revendication 1, comprenant en outre la mesure d'une ou plusieurs valeurs d'intensité du signal observé lors des étapes d'observation b, f, j ou d'une de leurs combinaisons.

15. Procédé selon la revendication 14, dans lequel la mesure d'une ou plusieurs valeurs d'intensité du signal comprend une technique d'amplification du signal.
